# FASCICULE DE BREVET EUROPEEN

(11) **EP 3 370 833 B1**
(45) Date de publication et mention de la délivrance du brevet: **04.12.2019**
(21) Numéro de dépôt: 16794748.0
(22) Date de dépôt: 04.11.2016
(51) Int. Cl.: A61Q 19/08, A61K 8/97, A61K 8/9706, A61K 8/9789, A61K 8/9717

(54) **EXTRAIT SYNERGIQUE DE PALMARIA PALMATA**
SYNERGISTISCHES EXTRAKT VON PALMARIA PALMATA
SYNERGISTIC EXTRACT OF PALMARIA PALMATA

(30) Priorité: 04.11.2015 FR 1560569
(43) Date de publication de la demande: 12.09.2018
(73) Titulaire: ISP Investments LLC, Wilmington, DE 19805 (US); LvmH Recherche, 45800 Saint-Jean De Braye (FR)
(72) Inventeur: LEQUOY, Valérie, 06560 Valbonne (FR); PORTOLAN, Frédérique, 06560 Valbonne (FR); LE MESTR, Audrey, 06600 Antibes (FR); CAPALLERE, Christophe, 06200 Nice (FR); IMBERT, Isabelle, 06400 Cannes (FR); MANTELIN, Joël, 06400 Cannes (FR); BOTTO, Jean Marie, 06560 Valbonne (FR); DOMLOGE, Nouha, 06650 Opio (FR); GARCIA, Noëlle, 06520 Magagnosc (FR); BERGERON, Laurine, 06600 Antibes (FR); NIZARD, Carine, 94200 Vitry-sur-Seine (FR)
(74) Mandataire: Cabinet Beau de Loménie
(86) Numéro de dépôt international: PCT/IB2016/056648
(87) Numéro de publication internationale: WO 2017/077497

(56) Documents cités:
- FR-A1- 2 826 575
- FR-A1- 2 999 425

## Description

### Domaine de l'invention

La présente invention se situe dans le domaine de la cosmétique et notamment pour le soin de la peau par une action au niveau du derme. Elle concerne plus particulièrement un extrait synergique d'une plante du genre *Jasminum* (encore appelée jasmin) et de l'algue rouge *Palmaria palmata,* un procédé d'obtention d'un tel extrait, des compositions comprenant un tel extrait et leurs utilisations cosmétiques pour lutter contre les signes du vieillissement de la peau, et notamment améliorer l'élasticité, la souplesse et la fermeté de la peau.

### Arrière-plan de l'invention

Le vieillissement correspond à l'ensemble des processus, notamment physiologiques, qui modifient la structure et les fonctions de l'organisme au cours du temps. On distingue deux types de vieillissement, à savoir d'une part, le vieillissement intrinsèque et, d'autre part, le vieillissement extrinsèque. Le vieillissement intrinsèque est dû à des facteurs génétiques, à des modifications biochimiques qui ont lieu lors d'états de fatigue, de stress, de changements hormonaux comme lors de la grossesse etc. Le vieillissement extrinsèque, quant à lui, est dû à des facteurs environnementaux auxquels est soumis l'organisme tout au long de sa vie, tels que la pollution, le soleil, les maladies etc. Il s'agit d'un processus lent et progressif qui atteint toutes les cellules de l'organisme par différents moyens et se manifeste de différentes manières. Par exemple, au niveau de la peau, l'aspect de celle-ci est modifié par les divers types d'agressions internes ou externes et l'on voit apparaître alors des rides et ridules, des taches d'hyper ou d'hypopigmentation, une sècheresse voire une déshydratation de la peau, un amincissement de l'épiderme, une élastose, des imperfections, des taches de vieillesse.

La peau est un organe de recouvrement composé principalement de trois couches cellulaires: l'épiderme, le derme, et l'hypoderme. L'épiderme, qui constitue la surface de la peau, est ancré au derme par une matrice de diverses protéines appelée jonction dermo-épidermique.

L'épiderme est constitué de plusieurs couches de cellules appelées kératinocytes, qui sont régénérées par les cellules couches de l'épiderme localisées dans la membrane basale de l'épiderme.

Le derme est le tissu de soutien de la peau et se compose majoritairement de cellules fibroblastes, de fibres d'élastine et de fibres de collagène (70 % des fibres dermiques), enveloppées dans une matrice extracellulaire interstitielle de protéoglycanes. Les fibroblastes sont à l'origine de la synthèse des fibres de collagène et de l'élastine. La néo-synthèse des fibres de tropoélastine, d'abord sous forme de proélastine, est un processus lié à l'activité des fibroblastes qui sécrètent ces fibres dans l'espace extracellulaire. Après maturation, l'élastine, associée à la fibrilline, représente la composante majeure des fibres élastiques qui confère au derme ses propriétés élastiques. D'autre part, les fibroblastes permettent de régénérer le tissu conjonctif et participent à la réparation de la peau après une blessure. Les fibroblastes qui sont impliqués dans de nombreuses fonctions au niveau de la peau, sont ainsi essentiels au maintien d'une peau saine et en bon état.

Les activités de renouvellement, ou de réparation des structures dermiques lors de dommages provoqués par exemple par les rayonnements UV ou les blessures, impliquent l'existence de cellules souches adultes dermiques (Skin Derived Precursors ou SKPs), localisées notamment dans les tissus tels que le prépuce et le follicule pilaire (Toma et al., 2005, Stem cells 23:727-737). Les cellules SKPs sont les principaux progéniteurs de cellules dermiques en ce qu'elles assurent le renouvellement des fibroblastes. Elles sont particulièrement mises en jeu lors de la réparation de blessures de la peau.

Les cellules SKPs possèdent des caractéristiques typiques des cellules souches en général; elles présentent des capacités d'auto-renouvellement et de différenciation importantes (Li et al., 2010, J Cell Sci. 123:853-60) et sont définies comme des cellules exprimant un ensemble de marqueurs moléculaires tels que:
- Nestin +: protéine de filament intermédiaire exprimée par de nombreuses cellules au cours du développement et en particulier les cellules de la crête neurale. Son expression est transitoire et ne persiste pas dans l'âge adulte.
- OCT4 + (octamère de liaison du facteur de transcription 4): marqueur de multipotence impliqué dans l'auto-renouvellement de cellules souches embryonnaires indifférenciées.
- SOX2 + (sexe région détermination Y-box 2): facteur de transcription essentiel pour maintenir l'auto-renouvellement des cellules souches embryonnaires indifférenciées.

Les recherches pour identifier des agents actifs capables de lutter contre le vieillissement cutané ont abouti à la mise sur le marché de nombreux agents actifs plus ou moins efficaces. Toutefois, il reste toujours d'actualité d'identifier de nouveaux composés capables de retarder l'apparition ou de lutter plus efficacement contre les signes du vieillissement cutané. Le problème plus particulièrement visé par l'invention est d'identifier de nouveaux agents actifs capables de lutter contre les principaux signes du vieillissement cutané siégeant au niveau de la matrice extracellulaire, dont la majorité des protéines la constituant est produite par les fibroblastes.

Les algues sont largement utilisées dans des applications cosmétiques. Leur potentiel à prévenir le vieillissement et à améliorer l'aspect et la protection de la peau humaine est connu. L'utilisation d'extraits d'algues peut améliorer la nutrition de la peau et des cheveux, tout en maintenant un bon taux d'hydratation.

Certaines espèces de micro-algues sont commercialisées pour les soins de la peau. Par exemple, des extraits *d'Arthrospira* et de *Chlorella* sont bien connus dans les crèmes anti-âge, les produits anti-irritants et les produits de soins rafraîchissants ou de régénération.

En outre, les espèces du genre d'algue *Palmaria,* en particulier l'espèce *Palmaria palmata,* sont connues pour être efficaces dans le soin de la peau. L'algue *Palmaria palmata* est encore appelée Dulce ou Dulse. Cette plante de la mer est riche en minéraux, en particulier le fluorure, le phosphore, le potassium, en vitamines, en protéines et en polysaccharides (xylanes). FR2826575 décrit l'utilisation cosmétique de xylanes extraits de *Palmaria palmata,* plus particulièrement pour augmenter l'hydratation de la couche cornée, mais aussi la régénération de la peau et des cheveux par synthèse de fibronectine et prolifération cellulaire fibroblastique.

Un extrait aqueux de fleurs de jasmin, en particulier de l'espèce *Jasminum officinale,* est une source connue de flavonoïdes aux propriétés antioxydantes. L'huile essentielle de jasmin est utilisée en aromathérapie (antioxydant) et en dermatologie (propriétés antiseptiques et anti-inflammatoires).

Toutefois, aucune des utilisations connues d'un extrait de *Jasminum officinale,* d'une part, et d'un extrait de *Palmaria palmata,* d'autre part, ne suggère que ces deux extraits présentent indépendamment l'un de l'autre, ou l'un en combinaison avec l'autre des propriétés i) d'augmentation de l'expression génique et/ou protéique du collagène, et/ou ii) d'augmentation du caractère souche des cellules couches dermiques.

Une des tendances de la cosmétique moderne est de développer des produits actifs d'origine naturelle, ayant non seulement des effets en tant qu'agents antirides ou antivieillissement mais aussi qui combinent plusieurs propriétés et apportent ainsi un spectre d'amélioration plus large des signes du vieillissement.

Or, les inventeurs ont mis en évidence qu'un nouvel extrait obtenu à partir de *Palmaria palmata* et de *Jasminum officinale* présente un grand intérêt pour les soins de la peau, en ce qu'il a un effet synergique sur les cellules souches dermiques. Un tel extrait permet en effet d'augmenter la réserve de cellules souches dermiques et améliore leur fonctionnement, ce qui permet de préserver et même de rétablir la structure du tissu conjonctif du derme. Un tel extrait permet d'améliorer les propriétés mécaniques de la peau, en particulier son élasticité, comme cela a été démontré par un test de ballistométrie, et de lutter ainsi contre certains signes du vieillissement cutané.

De manière surprenante, les inventeurs ont découvert que l'extrait synergique de *Palmaria palmata* et de sommités fleuries de jasmin utilisé selon l'invention offre notamment les avantages suivants :
- il augmente la quantité de marqueurs biochimiques associés au caractère « souche » des cellules souches dermiques (SKPs) et en particulier la Nestin +, OCT4 +, SOX2 + dans les cellules dermiques ;
- il augmente l'élasticité de la peau, la souplesse de la peau et/ou la fermeté de la peau,
- il augmente le renouvellement de la peau ;
- il augmente la synthèse de protéines de la matrice extracellulaire dermique, par exemple le collagène, en particulier le collagène I, III ou V ; le procollagène, en particulier le procollagène I, III ou V ; et la tropoélastine ;
- il permet en conséquence de lutter contre les signes cutanés liés au vieillissement de la peau.

On entend par « caractère souche des cellules souches dermiques», le profil d'expression de marqueurs biochimiques associés au phénotype des cellules SKPs ou SKP-like, en particulier l'expression Nestin +, OCT4 +, SOX2 +.

L'invention et les avantages qui en découlent seront mieux compris à la lecture de la description.

### Exposé de l'invention

La présente invention concerne un extrait d'une plante du genre *Jasminum* obtenu par macération d'au moins une partie de la plante dans un hydrolysat aqueux de l'algue *Palmaria palmata.* Le ratio massique entre le poids sec de l'algue et le poids sec de la plante, toutes deux utilisées comme matière première pour préparer l'extrait est de préférence compris entre 40/60 et 95/5. Cet extrait présente l'originalité de ne pas avoir des propriétés biologiques identiques à celles que l'on obtient avec le mélange d'un hydrolysat aqueux de l'algue *Palmaria palmata,* et d'un extrait d'une plante du genre *Jasminum* obtenu par macération d'au moins une partie de la plante dans de l'eau.

La présente invention a pour premier objet un extrait synergique de l'algue *Palmaria palmata* et d'au moins une partie d'une plante du genre *Jasminum,* lequel extrait est susceptible d'être obtenu par un procédé comprenant i) une étape de préparation d'un extrait aqueux de l'algue *Palmaria palmata* suivie ii) d'une étape de macération d'au moins une partie d'une plante du genre *Jasminum* dans ledit extrait aqueux, le ratio massique entre le poids sec de l'algue et le poids sec de la partie de la plante, toutes deux utilisées comme matière première pour préparer l'extrait synergique, étant compris entre 40/60 et 95/5.

Dans un mode de réalisation, l'extrait synergique est obtenu par ce procédé.

Dans la présente invention, les pourcentages sont exprimés en poids/poids, sauf indication contraire.

Dans la suite de la description, on utilise indifféremment les termes « jasmin » et «plante du genre *Jasminum* ».

Le terme "extrait" désigne de manière générale une substance isolée, obtenue à partir d'une matière première végétale native, et qui ne préexiste pas dans la nature en tant que tel.

Dans la présente description, la mention de l'algue ou de la plante, s'entend de la matière végétale récoltée, éventuellement séchée (par toute méthode connue, comme un séchage en étuve ou une lyophilisation), et éventuellement réduite en poudre ou en paillettes par broyage.

On entend par « extrait synergique» selon l'invention, un extrait comprenant ou constitué d'un extrait aqueux de *Palmaria palmata* et de jasmin, de préférence de sommités fleuries de *Jasminum officinale,* capable d'augmenter l'expression de Nestin +, OCT4 +, SOX2 +, soit en augmentant la synthèse protéique par modulation directe ou indirecte de l'expression génique, soit par d'autres processus biologiques tels que la stabilisation de la protéine ou encore la stabilisation des transcrits d'ARN messager, par rapport à un extrait de *Palmaria palmata* de référence incubé seul, à un extrait de jasmin de référence incubé seul, et à leur mélange. Un extrait de *Palmaria palmata* de référence pourra par exemple être obtenu par mise en œuvre de l'étape i) du procédé de préparation de l'extrait synergique tel que décrit précédemment, dans des conditions de préparation identiques. Un extrait de jasmin de référence pourra par exemple être obtenu par mise en œuvre de l'étape ii) du procédé de préparation de l'extrait synergique tel que décrit précédemment, dans des conditions de préparation identiques en remplaçant la masse d'extrait aqueux de *Palmaria* obtenue à l'issue de l'étape i), par la même masse d'eau. Dans toute la description, l'augmentation des propriétés est évaluée à poids constant en matière sèche d'extrait.

En particulier, un extrait synergique selon l'invention est un extrait comprenant ou constitué d'un extrait aqueux de *Palmaria palmata* et de sommités fleuries de jasmin capable de multiplier au moins par 2 le caractère souche de cellules souches dermiques, par rapport à un extrait de *Palmaria palmata* de référence incubé seul, à un extrait de jasmin de référence incubé seul, et éventuellement à un de leurs mélanges.

En particulier, l'extrait synergique peut être capable:
- de multiplier au moins par 8 la quantité d'ARN messager de SOX2 + exprimée par des fibroblastes, et/ou
- de multiplier au moins par 2 la quantité d'ARN messager de Nestin + exprimée par des fibroblastes, et/ou
- de multiplier au moins par 6 la quantité d'ARN messager de OCT4 + exprimée par des fibroblastes,
par rapport à un extrait de *Palmaria palmata* de référence incubé seul, et à un extrait de jasmin de référence incubé seul.

Les termes « extrait synergique », « extrait synergique de *Palmaria palmata* et de sommités fleuries de jasmin » ou « agent actif » seront utilisés alternativement avec le même sens dans le cours la description.

La partie de la plante du genre Jasminum peut être la racine, la tige, les feuilles, les fleurs ou les graines. On préfère que ladite partie comprenne les fleurs. On entend par « sommités fleuries », une partie de la plante comprenant la fleur éventuellement accompagnée de tige. Dans un mode de réalisation, les sommités fleuries comprennent la fleur et quelques centimètres de tige.

L'extrait selon l'invention est préférentiellement obtenu après une extraction aqueuse de *Palmaria palmata* dans laquelle est ensuite réalisée une macération des sommités fleuries de jasmin, le ratio massique entre le poids sec de l'algue et le poids sec des sommités fleuries, allant de 50/50 et 90/10, par exemple de 60/40 à 70/30 ou de 80/20 à 90/10 (les bornes étant comprises). Dans un mode de réalisation, le ratio massique est égal à 90/10.

La préparation de l'extrait peut débuter par la préparation d'un extrait aqueux de *Palmaria palmata,* qui est une espèce d'algues rouges de la famille des Palmariaceae encore appelée Dulce ou Dulse. Elle a été une source importante de fibres à travers les siècles. Cette algue est riche en minéraux, en particulier le fluorure, le phosphore, le potassium, minéraux, vitamines, protéines et polysaccharides (xylanes).

L'extrait aqueux de *Palmaria palmata* utilisé selon l'invention peut être obtenu, par hydrolyse enzymatique, par exemple avec une carbohydrase et/ou une endoprotéase, d'une solution aqueuse de *Palmaria palmata* comprenant un rapport massique eau / *Palmaria palmata* (exprimé en poids sec de l'algue), compris entre 10/1 et 50/1, à un pH compris entre 3 et 6, à une température comprise entre 40 et 80°C, pendant un temps d'au moins 1 heure, préférentiellement 2 heures.

Les algues de *Palmaria palmata* sont avantageusement séchées et finement broyées, après leur récolte.

Le rapport massique eau / *Palmaria palmata* est préférentiellement compris entre 15/1 et 30/1, encore plus préférentiellement entre 20/1 et 25/1.

Le pH est préférentiellement ajusté, par exemple par ajout d'acide chlorhydrique (HCl), entre 3 et 6, préférentiellement entre 4 et 5,5, encore plus préférentiellement entre 4 et 4,5.

La température d'hydrolyse est préférentiellement comprise entre 40°C et 80°C, préférentiellement entre 50 et 60°C et encore plus préférentiellement de 55 °C.

L'utilisation d'extraits de plantes hydrolysés présente de nombreux avantages en cosmétique et dermo-cosmétique. Outre le fait de libérer des composés actifs, l'hydrolyse et la purification permettent d'obtenir des mélanges plus stables, plus facilement standardisables et ne provoquant pas de réactions allergiques en cosmétique.

De façon avantageuse, l'hydrolyse contrôlée permet l'accès aux sucres contenus dans les algues de l'espèce *Palmaria palmata.* L'extrait selon l'invention est un extrait aqueux de jasmin et *Palmaria* enrichi en composés d'intérêt de *Palmaria palmata* et du jasmin.

L'hydrolyse enzymatique contrôlée est préférentiellement réalisée par une xylanase, en tant que carbohydrase, et une bromélaïne, en tant qu'endoprotéase. Ces enzymes permettent d'optimiser le rendement et le taux d'hydrolyse.

Les xylanases sont des enzymes du groupe des glycosyles hydrolases qui catalysent l'hydrolyse de β-1,4-glucosidiques en xylane via un double mécanisme de déplacement. L'hydrolyse des xylanes libère du xylose.

Préférentiellement, l'endoprotéase utilisée dans le procédé selon l'invention est la bromélaïne, également appelée bromélase. Il s'agit d'une enzyme protéolytique extraite des tiges et racines fraîches de l'ananas. C'est un mélange d'enzymes à action protéolytiques, qui cible les groupements sulfatés des chaines latérales des cystéines.

La xylanase est utilisée en une quantité comprise préférentiellement entre 2 et 6%, encore plus préférentiellement 4% par rapport à la quantité en poids sec d'algue introduite dans le milieu réactionnel et la bromélaïne en une quantité préférentiellement comprise entre 1 et 3%, encore plus préférentiellement 2%.

L'extrait aqueux de *Palmaria palmata* ainsi obtenu est ensuite séparé des résidus solides par une méthode connue de l'homme de métier telle qu'une centrifugation suivie d'une filtration.

C'est ce premier extrait aqueux filtré qui servira de liquide de macération pour les sommités fleuries de jasmin, par exemple.

La macération désigne un procédé qui consiste à laisser séjourner pendant un temps déterminé un solide dans un liquide pour en extraire les composés solubles.

Préférentiellement, la macération est réalisée pendant un temps d'au moins 2 heures et jusqu'à 4 heures à température ambiante, par exemple à une température comprise entre 18 et 35°C.

*Jasminum officinale* ou jasmin blanc (ou jasmin officinal) est un arbuste grimpant, de la famille des Oleaceae, au feuillage caduc à semi-persistant, donnant une abondante floraison parfumée durant tout l'été.

Des sommités fleuries de jasmin (qui comprennent comme partie de la plante, la fleur et sont accompagnées de quelques centimètres de tige) sont préférentiellement choisies parmi les sommités fleuries de l'une des espèces *Jasminum grandiflorum, Jasminum officinale, Jasminum odoratissimum, Jasminum sambac, Jasminum auriculatum, Jasminum flexile,* préférentiellement *Jasminum officinale.* Le jasmin appartient de préférence à l'espèce *Jasminum officinale.*

Les sommités fleuries de jasmin sont avantageusement utilisées entières et séchées et laissées macérer dans l'extrait aqueux de *Palmaria palmata.*

L'extrait synergique de *Palmaria palmata* et de sommités de sommités fleuries de jasmin ainsi obtenu, après filtration, a une teneur en matière sèche comprise entre 26,8 et 30,8 g/kg, une concentration en protéines comprise entre 1,3 et 2,3 g/kg et une concentration en sucres (dont une majorité de xylose) comprise entre 25,3 et 29,3 g/kg.

L'extrait peut ensuite être dilué dans un ou plusieurs solvants physiologiquement acceptables tels que l'eau, le glycérol, l'éthanol, le propanediol, le butylène glycol, le dipropylène glycol, les diglycols éthoxylés ou propoxylés, les polyols cycliques ou tout mélange de ces solvants. Préférentiellement, l'extrait est dilué dans de l'eau et du xylitol de façon à obtenir un extrait final à 30% en poids de xylitol. L'extrait synergique selon l'invention est alors caractérisé par une concentration en matière sèche comprise entre 280 et 320 g/kg, une concentration en sucres comprise entre 8 et 12 g/kg, et un pH compris entre 4 et 5.

Un deuxième objet de l'invention concerne un procédé d'obtention d'un extrait synergique de *Palmaria Palmata* et de sommités fleuries de jasmin, comprenant les étapes suivantes selon lesquelles :
- on met en solution dans de l'eau une quantité de *Palmaria palmata* dans un rapport massique eau / *Palmaria palmata* (en poids sec) compris entre 10/1 et 50/1 ;
- on hydrolyse la solution aqueuse de *Palmaria palmata* par une carbohydrase et par une endoprotéase, à une température comprise entre 40 et 80°C;
- on fait macérer des sommités fleuries de jasmin dans l'extrait aqueux de *Palmaria palmata* obtenu précédemment; le ratio massique entre le poids sec de l'algue et le poids sec des sommités fleuries, étant compris entre 40/60 et 95/5.
- on filtre le macérât obtenu puis on le chauffe pendant 2 à 24 heures, à une température comprise entre 40 et 90°C, pour désactiver les enzymes carbohydrase et endoprotéase.

Selon un mode de réalisation particulier, le procédé d'obtention d'un extrait synergique de *Palmaria Palmata* et de sommités fleuries de jasmin selon l'invention comprend les étapes suivantes selon lesquelles :
a) on met en solution dans de l'eau une quantité de *Palmaria palmata* séchées et finement broyées sous forme de paillettes dans un rapport massique eau / *Palmaria palmata* compris entre 10/1 et 50/1, préférentiellement entre 20/1 et 40/1.
b) on hydrolyse la solution aqueuse de *Palmaria palmata* par une carbohydrase et une endoprotéase, préférentiellement réalisée par une xylanase et une bromélaïne, à un pH compris entre 3 et 6, préférentiellement entre 4 et 5,5, encore plus préférentiellement entre 4 et 4,5, à une température comprise entre 40 et 80°C, préférentiellement entre 50 et 60°C, encore plus préférentiellement 55°C, pendant un temps d'au moins 1 heure, préférentiellement 2 heures ;
c) après ajout éventuel d'un adjuvant de filtration et d'une centrifugation, on obtient un extrait aqueux de *Palmaria Palmata* ;
d) on fait macérer pendant un temps d'au moins 2 heures au plus 4 heures à température ambiante des sommités fleuries séchées de jasmin dans l'extrait aqueux de *Palmaria palmata* obtenu à l'étape c) ; le ratio massique entre le poids sec de l'algue et le poids sec des sommités fleuries, étant compris entre 40/60 et 95/5. Préférentiellement le ratio massique entre le poids sec de l'algue et le poids sec des sommités fleuries est égal à 90/10 ;
e) on filtre le macérât ainsi obtenu à l'étape d) pour récupérer un extrait de *Palmaria palmata* et de sommités fleuries de jasmin que l'on chauffe pendant au moins 2 heures et jusqu'à 24 heures et préférentiellement pendant 12 heures ou une nuit, à une température comprise entre 40 et 90°C, préférentiellement à 80°C pour désactiver les enzymes carbohydrase et endoprotéase ; et
f) on purifie éventuellement par filtration pour obtenir l'extrait synergique de *Palmaria palmata* et de sommités fleuries de Jasmin.

Au cours de l'étape d), les sommités fleuries de jasmin ne sont pas nécessairement mises à macérer dans un milieu contenant un glycol ou plus généralement un alcool comme le méthanol ; on préfère utiliser un milieu liquide à base d'eau uniquement. De plus, l'étape de macération des fleurs dans l'extrait aqueux de *Palmaria* ne comprend pas nécessairement une étape d'hydrolyse enzymatique, qui est parfois envisagée dans l'art antérieur pour extraire des fleurs les composés phénoliques et glucidiques qu'elles contiennent. Une hydrolyse enzymatique est réalisée sur l'extrait aqueux de *Palmaria,* avant l'ajout des fleurs de jasmin.

Après les étapes b) et e), la solution obtenue peut être trouble. Pour éliminer les résidus solides en suspension, des étapes de centrifugation et de filtration sont réalisées. Un adjuvant de filtration tel que le Celatom® peut être ajouté au mélange, puis une étape de filtration est réalisée pour séparer les solides de la phase liquide, les solides étant éliminés. Plusieurs étapes de filtrations successives sur filtres de porosité décroissante peuvent ensuite être effectuées. Le filtrat recueilli constitue l'extrait de *Palmaria palmata* riche en polysaccharides.

Le filtrat résultant de l'hydrolyse enzymatique de *Palmaria palmata* et de la macération de sommités fleuries de jasmin dans l'extrait de *Palmaria,* après désactivation des enzymes résiduelles constitue une première forme de l'extrait synergique actif selon l'invention, ou premier filtrat. A ce stade, le premier filtrat a par exemple une concentration en matière sèche comprise entre 26,8 à 30,8 g/kg, un contenu en composés protéiques compris entre 1,3 et 2,3 g/kg et un contenu en sucre compris entre 25,3 et 29,3 g/kg.

Ce premier filtrat actif peut être alors dilué dans un ou plusieurs solvants physiologiquement acceptables, tels que l'eau, le glycérol, l'éthanol, le propanediol, le butylène glycol, le dipropylène glycol, les diglycols éthoxylés ou propoxylés, les polyols cycliques ou tout mélange de ces solvants. Dans un mode de réalisation préféré, le premier filtrat actif est dilué dans un mélange de solvant tel que l'on obtienne un deuxième filtrat ou extrait final contenant 30% de xylitol. L'extrait synergique selon l'invention peut également être préservé des contaminations avec du benzoate de sodium à 0,5%.

Le deuxième filtrat dilué dans un solvant peut être ensuite filtré sous conditions stériles, pasteurisé à basse température, de préférence à 65°C toute une nuit, pour compléter la stérilisation.

Selon ce mode de mise en œuvre, on obtient un filtrat final qui constitue l'extrait synergique selon l'invention. L'extrait synergique obtenu selon l'invention peut être analysé qualitativement et quantitativement, selon les techniques classiques bien connues de l'homme du métier, pour déterminer ses caractéristiques physico-chimiques et sa teneur en composés.

Préférentiellement, lorsque le premier filtrat actif contient 30% de xylitol, l'extrait synergique selon l'invention est caractérisé par une concentration en matière sèche comprise entre 280 et 320 g/kg, une concentration en sucres comprise entre 8 et 12 g/kg, et un pH compris entre 4 et 5.

Le troisième objet de la présente invention est une composition comprenant, dans un milieu physiologiquement acceptable, pour lutter contre les signes du vieillissement cutané, l'extrait synergique selon l'invention, à une concentration comprise entre 0,0001 % et 20 % en poids sec du poids total de la composition, et préférentiellement à une concentration comprise entre 0,05 % et 5 % en poids sec du poids total de la composition.

Par « physiologiquement acceptable », on entend que l'extrait synergique selon l'invention, ou une composition contenant ledit agent, est adapté pour venir en contact avec la peau ou une membrane muqueuse, sans provoquer une réaction de toxicité ou d'intolérance.

L'extrait synergique selon l'invention peut être encapsulé ou inclus dans un vecteur cosmétique ou pharmaceutique tels que les liposomes ou toute autre microcapsule utilisée dans le domaine de la cosmétique ou adsorbé sur des polymères organiques poudreux, des supports minéraux comme les talcs et bentonites.

Ces compositions pourront notamment se présenter sous forme d'une solution aqueuse, hydro-alcoolique ou huileuse ; d'une émulsion huile-dans-eau, eau-dans-huile ou émulsions multiples ; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick, ou être appliquées sur la peau sous forme d'aérosol. Elles peuvent être utilisées comme produit de soin et/ou comme produit de maquillage de la peau.

Ces compositions comprennent, en outre, tout additif communément utilisé dans le domaine d'application envisagé ainsi que les adjuvants nécessaires à leur formulation, tels que des solvants, des co-solvants (éthanol, le glycérol, l'alcool benzylique), des épaississants, des diluants, des antioxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, des oligo-éléments, des polymères filmogènes, des filtres chimiques ou minéraux, des agents hydratants ou des eaux thermales, des polymères tels que des polysaccharides ou des polypeptides, des dérivés de cellulose de la méthylcellulose type ou l'hydroxypropylcellulose, ou encore des polymères synthétiques, les poloxamères, les carbomères, les siloxanes, PVA ou PVP.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

La composition utilisable selon l'invention pourra être appliquée par toute voie appropriée, notamment orale ou topique externe, et la formulation des compositions sera adaptée par l'homme du métier.

Avantageusement, les compositions selon l'invention se présentent sous une forme adaptée à une application par voie topique. Ces compositions doivent donc contenir un milieu physiologiquement acceptable, c'est-à-dire compatible avec la peau et les phanères, et couvrent toutes les formes cosmétiques.

Avantageusement, la composition utilisable pour réaliser l'invention peut comprendre, outre l'agent actif selon l'invention, au moins un autre agent actif présentant des effets cosmétiques similaires et/ou complémentaires à ceux de l'invention. Selon l'invention, cet agent actif sera défini comme un « agent actif additionnel ».

Par exemple, le ou les agents actifs additionnels peuvent être choisi parmi : les agents anti-âges, raffermissants, éclaircissants, hydratants, drainants, favorisant la microcirculation, agents pharmaceutiques, exfoliants, desquamants, stimulant la matrice extracellulaire, activant le métabolisme énergétique, antibactériens, antifongiques, apaisants, anti-radicalaires, anti-UV, anti-acnés, anti-inflammatoires, anesthésiques, procurant une sensation de chaleur, procurant une sensation de fraicheur, amincissants.

De tels agents additionnels peuvent être choisis dans les groupes comprenant :
- la vitamine A et notamment l'acide rétinoïque, le rétinol, le rétinol proprionate, le rétinol palmitate,
- la vitamine B3 et plus particulièrement le niacinamide, le niconitate de tocophérol,
- la vitamine B5, la vitamine B6, la vitamine B12, le panthénol,
- la vitamine C, notamment l'acide ascorbique, l'ascorbyl glucoside, l'ascorbyl tétrapalmitate, magnésium et sodium ascorbyl phosphate,
- les vitamines E, F, H, K, PP, le coenzyme Q10,
- les inhibiteurs de métalloproteinase, ou un activateur des TIMP,
- la DHEA, ses précurseurs et dérivés,
- les acides aminés tels que l'arginine, ornithine, hydroxyproline, hydroxyproline dipalmitate, palmitoylglycine, hydroxylysine, méthionine et ses dérivés, composés acides aminés N-acyl,
- les peptides naturels ou de synthèse, incluant les, di-, tri-, tétra-, penta- et hexapeptides et leurs dérivés lipophiles, isomères et complexés avec d'autres espèces telles qu'un ion métal (e.g. cuivre, zinc, manganèse, magnésium, et autres). Par exemple les peptides commercialement connus sous le nom MATRIXYL®, ARGIRELINE®, COLLAXYL™, PEPTIDE VINCI 02™, CHRONOGEN™, LAMINIXYL IS™, PEPTIDE Q10™, le peptide de synthèse de séquence Arg-Gly-Ser-NH₂, commercialisé sous le nom ATPeptide™, le peptide de synthèse de séquence Pro-Leu-Asp-Thr-Ala-Lys-Val-Arg-Leu-Gln commercialisé sous le nom SIRPeptide™.
- les extraits peptidiques végétaux tels que extraits de soja, d'épeautre, de vigne, de colza, de lin, de riz, de maïs, de pois,
- les extraits de levures, les extraits *d'Artemia Salina*,
- l'acide déhydroacétique (DHA),
- les phystostérols d'origine synthétique ou naturelle,
- l'acide salicylique et ses dérivés, les alpha- et bêta-hydroxyacides, les silanols,
- les sucres aminés, glucosamine, D-glucosamine, N-acetyl- glucosamine, N-aceétyl-D-glucosamine, mannosamine, N-acetyl mannosamine, galactosamine, N-acetyl galactosamine,
- les extraits de polyphénols, isoflavones, flavonoïdes, tels que les extraits de raisin, les extraits de pin, les extraits d'olives,
- les lipides tels que les céramides ou les phospholipides, les huiles d'origine animale, telles que le squalène ou le squalane ; les huiles végétales, telles que l'huile d'amandes douces, de coprah, de ricin, de jojoba, d'olive, de colza, d'arachide, de tournesol, de germes de blé, de germes de maïs, de soja, de coton, de luzerne, de pavot, de potiron, d'onagre, de millet, d'orge, de seigle, de carthame, de passiflore, de noisette, de palme, de noyau d'abricot, d'avocat, de calendula ; les huiles végétales éthoxylées, le beurre de karité,
- les écrans UV et filtres solaires.

Un autre aspect de l'invention concerne l'utilisation cosmétique d'un extrait synergique de *Palmaria palmata* et de sommités fleuries de jasmin selon l'invention, pour lutter contre les signes de vieillissement de la peau, en favorisant notamment le maintien du caractère « souche » des cellules souches adultes dermiques SKPs.

L'invention s'adresse aux mammifères et aux êtres humains en particulier.

Par "signes du vieillissement cutané", on entend toutes modifications de l'aspect extérieur de la peau et des phanères dues au vieillissement intrinsèque et extrinsèque comme par exemple les rides et ridules, le flétrissement, la perte de fermeté, l'amincissement, le manque d'élasticité et/ou de tonus, le ternissement et la perte d'éclat, mais également toute modification interne de la peau qui ne se traduit pas systématiquement par un aspect extérieur modifié comme, par exemple, toute dégradation interne de la peau consécutive aux agressions externes telles que le rayonnement ultraviolet (UV). L'agent actif selon l'invention, ou la composition le contenant, permettra de lutter, en particulier, contre la perte d'élasticité, de souplesse et de fermeté de la peau.

Par « lutter contre les signes du vieillissement cutané » on entend retarder l'apparition, réduire, améliorer l'aspect visuel, ou encore corriger de tels signes. On entend en particulier, par « lutter contre les signes du vieillissement » améliorer les propriétés mécaniques de la peau, notamment augmenter sa fermeté, sa souplesse et/ou son élasticité.

Aussi, l'invention concerne encore l'utilisation cosmétique d'un extrait synergique de *Palmaria palmata* et de sommités fleuries de jasmin pour améliorer l'élasticité de la peau.

L'invention concerne encore l'utilisation cosmétique d'un extrait synergique de *Palmaria palmata* et de sommités fleuries de jasmin pour améliorer les propriétés élastiques de la peau.

Le terme «peau» selon l'invention englobe la peau glabre et l'ensemble des annexes kératiniques présentes à la surface du corps, en particulier les poils, les cils, les sourcils, les ongles et les cheveux.

Le renouvellement permanent de la peau, mais aussi sa réparation après un dommage tel que les rayonnements UV ou les blessures, impliquent l'existence de cellules souches somatiques appelées cellules souches dermiques ou SKPs dans la peau. Cette réserve de cellules souches diminue lors vieillissement et suite aux agressions extérieures, et ce faisant la capacité de renouvellement de la peau diminue et contribue à l'apparition de signes de vieillissement de la peau.

On entend par l'expression « agression extérieure », les agressions que peut produire l'environnement. A titre d'exemple, on peut citer des agressions telles que la pollution, les rayonnements ultraviolets, ou encore les produits à caractère irritant tels que les tensioactifs, les conservateurs ou les parfums. Par pollution, on entend aussi bien la pollution « extérieure » due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldéhyde), ou bien encore la fumée de cigarette. La sécheresse de l'atmosphère est également une cause importante de desséchement cutané.

Or, les inventeurs ont trouvé que l'agent actif selon l'invention, d'une manière surprenante, protège les cellules SKPs et renforce leur activité. En effet, l'extrait selon l'invention permet une augmentation synergique des marqueurs caractéristiques du caractère « souche » des cellules souches dermiques, très supérieure à l'activation observée après le traitement par un extrait de *Palmaria palmata* seul ou par un extrait aqueux de sommités fleuries de jasmin seul.

L'extrait synergique pourra ainsi être utilisé, en tant qu'agent actif, dans une composition cosmétique pour prévenir ou réparer les dommages causés à la peau par le vieillissement, éventuellement accéléré par une exposition au soleil ou un environnement desséchant.

Ainsi, un aspect de l'invention concerne l'utilisation cosmétique d'un extrait de sommités fleuries de jasmin obtenu par macération desdites sommités dans un extrait aqueux de *Palmaria palmata,* pour améliorer au moins une des conditions d'une zone de peau saine d'une personne, choisies dans le groupe consistant en la fermeté de la peau, l'élasticité de la peau, la souplesse de la peau et la vitesse de renouvellement de la peau.

Au sens de la présente invention, on entend par « utilisation cosmétique », une utilisation qui n'est pas destinée à un usage thérapeutique. Dans le cadre de cette utilisation, l'extrait est appliqué sur une partie de la peau d'une personne qui est saine. Une zone de peau saine est facilement caractérisable par un dermatologue, qui n'y décèle aucune maladie, aucune affection cutanée (psoriasis, eczéma ou acné) et aucune plaie.

Au cours du vieillissement, la quantité de collagène diminue globalement, ce qui entraîne une perte de fermeté de la peau. Ce phénomène peut être accéléré lorsque la peau est soumise à des agressions extérieures telles que le froid ou les UV.

Le vieillissement de la peau se manifeste par différents signes qui ont des causes biologiques indépendantes les unes des autres, si bien qu'il est possible de lutter contre le vieillissement de la peau en proposant des actifs qui interviennent sur des cibles particulières pour induire la diminution d'un signe du vieillissement particulier.

Parmi ces manifestations figurent la perte de fermeté, l'augmentation de la rigidité et la perte d'élasticité. La principale cause de dégradation des propriétés mécaniques de la peau repose sur la diminution de la quantité de collagène dans la matrice extracellulaire dermique. Cette diminution revêt elle-même des causes différentes et indépendantes les unes des autres, parmi lesquels la dégradation des molécules de collagène par des enzymes, la glycation de ces mêmes molécules, et la diminution de la production de collagène par les fibroblastes.

Des actifs biologiques à effet anti-vieillissement connus de l'homme du métier que l'on incorpore dans des compositions cosmétiques inhibent l'activité des collagénases, responsables de la dégradation du collagène. D'autres actifs qui inhibent le mécanisme de glycation du collagène ont également été proposés dans des produits de soin cosmétique. Ces actifs et ces compositions cosmétiques ralentissent la dégradation et la désorganisation des fibres de collagène. De tels produits ne permettent d'augmenter ni la quantité de protéines dermiques neosynthétisées, ni le taux de renouvellement cellulaire.

Il subsiste donc le besoin de proposer des actifs biologiques plus efficaces pour lutter contre les signes du vieillissement cutané. Le but de la présente invention est de fournir un nouvel extrait d'origine végétale qui permet non pas de stabiliser la quantité de collagène dans la peau au cours du temps, mais d'augmenter l'expression génique et/ou protéique du collagène, et par conséquent d'améliorer les propriétés mécaniques de la peau, telles que la souplesse et l'élasticité.

Selon un de ses aspects, l'invention concerne l'utilisation de l'extrait décrit précédemment ou préparé selon le procédé décrit précédemment pour lutter contre le vieillissement de la peau, par augmentation de son niveau de souplesse, de son niveau d'élasticité, et/ou de sa vitesse de renouvellement cellulaire.

L'augmentation de la souplesse et de l'élasticité de la peau produite par l'extrait de l'invention peut être liée à l'augmentation de l'expression d'au moins une protéine de la matrice extracellulaire du derme choisie parmi le collagène, la tropoélastine et le procollagène. L'augmentation de l'expression d'une protéine dans le derme peut correspondre à l'augmentation de l'expression génique et/ou de l'expression protéique de ladite protéine. Dans un mode de réalisation particulier, l'augmentation de la fermeté et de l'élasticité de la peau est causée par les augmentations simultanées de la synthèse de collagène et d'élastine, par exemple par augmentation simultanée des synthèses de collagène I, de collagène III, de procollagène I, de procollagène III, et de tropoélastine.

L'augmentation du renouvellement cellulaire de la peau peut se traduire par l'augmentation du nombre de cellules souches dermiques et/ou par l'amélioration de leur fonctionnement, ce qui permet de rétablir la structure du tissu conjonctif dermique qui était détériorée avant l'application de l'extrait synergique. Dans un cas particulier, l'invention vise à restaurer ou augmenter le caractère souche des cellules souches dermiques en augmentant l'expression de l'un au moins des marqueurs Nestin +, OCT4 + et SOX2 + exprimés par des fibroblastes.

L'invention a plus particulièrement comme objet l'utilisation cosmétique d'un extrait de sommités fleuries de jasmin obtenu par macération desdites sommités dans un extrait aqueux de Palmaria palmata, pour lutter contre le vieillissement de la peau en augmentant la fermeté, la souplesse et/ou l'élasticité de la peau. L'augmentation de la fermeté, de la souplesse et/ou de l'élasticité peut être induite par l'augmentation de la synthèse de protéines par les fibroblastes dans le derme.

L'invention a également comme objet l'utilisation cosmétique d'un extrait de sommités fleuries de jasmin obtenu par macération desdites sommités dans un extrait aqueux de *Palmaria palmata,* pour augmenter le renouvellement cellulaire du derme en augmentant notamment le caractère souche des cellules souches dermiques (SKPs) et/ou en augmentant la quantité de cellules souches dermiques dans la peau.

La présente invention concerne également l'utilisation cosmétique, avantageusement par voie topique, de l'extrait décrit précédemment, pour augmenter l'expression d'au moins une protéine du derme. L'augmentation de l'expression d'une protéine dans le derme peut correspondre à l'augmentation de l'expression génique et/ou de l'expression protéique de ladite protéine. Cette protéine est par exemple choisie parmi le collagène, la tropoélastine et le procollagène. Le collagène est de préférence le collagène I ou le collagène III. Le procollagène est de préférence le procollagène I, ou le procollagène III. Dans un mode de réalisation particulier, l'invention concerne l'utilisation de l'extrait pour augmenter de façon simultanée l'expression protéique de procollagène I, l'expression protéique de collagène III, et l'expression protéique de tropoélastine.

L'augmentation de l'expression du collagène du derme peut être une augmentation du niveau d'expression protéique du collagène d'au moins 15 % par rapport au niveau d'expression protéique du collagène mesuré en l'absence de l'extrait selon l'invention. Cette augmentation est de préférence mesurée dans des équivalents de derme contenant des fibroblastes et des cellules souches dermiques de type SKP ou SKP-like obtenues à partir de fibroblastes de donneur adulte, en utilisant une quantité d'extrait sec en poids de l'ordre de 0,01 % du poids de l'équivalent de derme. Par exemple, il s'agit d'une augmentation du niveau d'expression protéique du procollagène I et du collagène III d'au moins 20 % dans des équivalents de derme, mesurée par exemple selon le protocole tel que décrit dans l'exemple 3, en utilisant une méthode d'immunomarquage révélée par observation avec un microscope à épifluorescence, éventuellement combinée à une augmentation du niveau d'expression protéique de la tropoélastine d'au moins 15 % dans des équivalents de derme, mesuré par exemple selon le protocole tel que décrit dans l'exemple 5, en utilisant une méthode d'immunomarquage révélée par observation avec un microscope à épifluorescence.

L'augmentation de la souplesse et/ou de l'élasticité peut être une augmentation d'au moins 15 % par rapport au niveau de souplesse ou respectivement d'élasticité d'un échantillon de peau mesuré avant application de l'extrait selon l'invention. L'augmentation de la souplesse et de l'élasticité peut être mesurée à l'aide d'un ballistomètre sur des équivalents de derme contenant des fibroblastes et des cellules souches dermiques de type SKP ou SKP-like, en utilisant une quantité d'extrait équivalant à un poids sec de l'ordre de 0,01% en poids du poids de milieu de culture.

La méthode de ballistométrie consiste à suivre les oscillations d'une bille que l'on laisse tomber d'une hauteur prédéterminée sur un échantillon de peau. La profondeur de pénétration de la bille à sa libération (nommée indentation) permet de mesurer la rigidité, et par conséquent la souplesse de la peau. En effet plus la bille s'enfonce profondément et plus sa rigidité est faible. L'élasticité est évaluée par calcul de la pente de la courbe (nommée alpha) reliant tous les sommets des pics d'oscillation. Plus une peau est élastique, plus la bille réalise de rebonds et plus la pente est faible.

L'augmentation de la souplesse, que l'on peut également exprimée comme la diminution de la rigidité, est par exemple une diminution de l'indentation d'au moins 15 % par rapport à l'indentation mesurée avant application de l'extrait selon l'invention, en utilisant une méthode de ballistométrie sur des équivalents de derme contenant des fibroblastes et des cellules souches dermiques de type SKP ou SKP-like, en particulier selon le protocole de l'exemple 6.

L'augmentation de l'élasticité est par exemple une augmentation d'au moins 15 % de la pente alpha par rapport à la pente alpha mesurée avant application de l'extrait selon l'invention, en utilisant une méthode de ballistométrie sur des équivalents de derme contenant des fibroblastes et des cellules souches dermiques de type SKP ou SKP-like, en particulier selon le protocole de l'exemple 6.

L'augmentation du renouvellement cellulaire de la peau peut être une augmentation de l'expression de l'un au moins des marqueurs Nestin, OCT4 et SOX2 dans des fibroblastes, mesurée par une méthode de PCR quantitative, en comparant la quantité des ARNm de SOX2, Nestin et/ou OCT4 exprimés par des cellules de fibroblastes cultivés in vitro, avant et après application de l'extrait de l'invention, utilisé en une quantité équivalant à un poids sec de l'ordre de 0,01% en poids du poids de milieu de culture. Les augmentations de l'expression des ARNm de SOX2, Nestin et OCT4 dans des fibroblastes sont de préférence respectivement d'au moins 250%, d'au moins 100% et d'au moins 250%.

L'invention a également pour objet une méthode de soin cosmétique consistant à appliquer sur la peau saine d'un sujet, un extrait synergique tel que décrit précédemment pour augmenter la fermeté, la souplesse et/ou l'élasticité de la peau, ou augmenter sa vitesse de renouvellement.

Les modes de réalisation qui sont spécifiques à ces méthodes de soin cosmétique et à ces utilisations résultent également de la description ci-dessus.

D'autres avantages et caractéristiques de l'invention peuvent être vus plus en détail, à la lecture des exemples illustratifs non limitatifs fournis.

Dans toutes les Figures, les valeurs numériques qui ont été utilisées pour la représentation graphiques sont les valeurs moyennes de trois mesures. Les barres d'erreurs correspondent aux valeurs calculées RQmin et RQmax, basées sur l'écart type moyen : ***: Hautement significatif; **: Très significatif, *: Significatif avec le test de Dunnett' s.

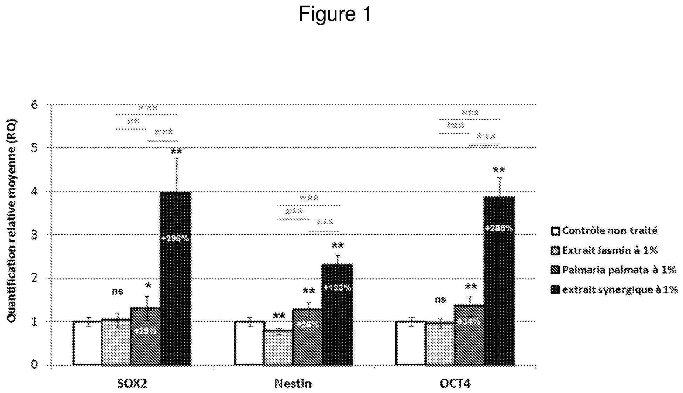

Figure 1 : qPCR des ARNm de SOX2, Nestin et OCT4 après application sur fibroblastes de différents extraits. Les valeurs numériques qui ont été utilisées pour la représentation graphiques sont les valeurs moyennes de trois mesures. Les barres d'erreurs correspondent aux valeurs calculées RQmin et RQmax, basées sur l'écart type moyen : ***: Hautement significatif; **: Très significatif, *: Significatif avec le test de Dunnett's.

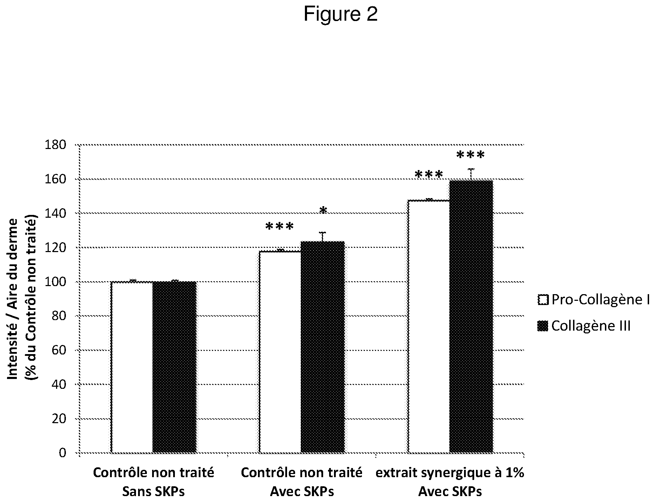

Figure 2 : Expression de protéines de la matrice extracellulaire (Procollagène I et Collagène III) après application de 1% de l'extrait synergique selon l'exemple 1 sur équivalent de derme contenant des cellules SKPs. Les valeurs numériques qui ont été utilisées pour la représentation graphiques sont les valeurs moyennes de trois mesures. Les barres d'erreurs correspondent aux valeurs calculées RQmin et RQmax, basées sur l'écart type moyen : ***: Hautement significatif; **: Très significatif, *: Significatif avec le test de Dunnett's.

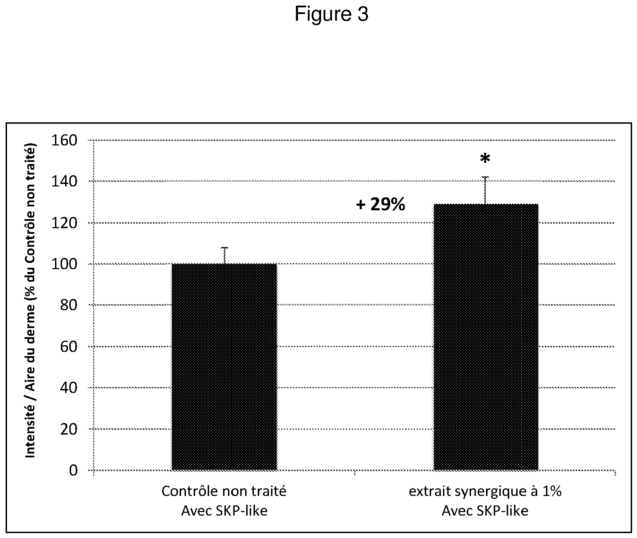

Figure 3 : Expression du collagène III après application de 1% de l'extrait synergique selon l'exemple 1 sur équivalent de derme contenant des cellules SKP-like. (moyenne+/- sem; n=6, 3 dermes par condition et 2 photos par derme équivalent). *: Significatif avec le test "t" de Student's - hypothèse unilatérale.

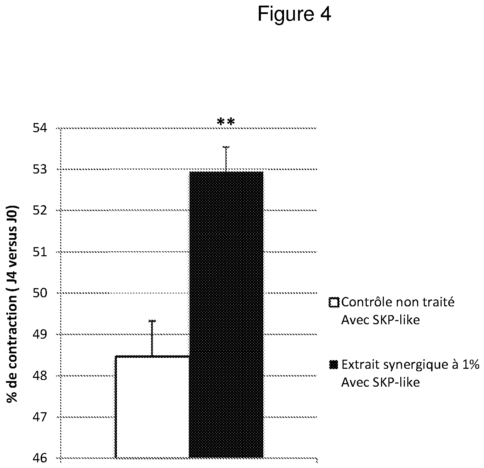

Figure 4 : Mesure de la contraction des équivalents de derme contenant des cellules SKP-like, après application de 1% de l'extrait synergique selon l'exemple 1. (moyenne+/- sem; n=3 derme équivalent). **: Très significatif avec le test "t" de Student's - hypothèse unilatérale.

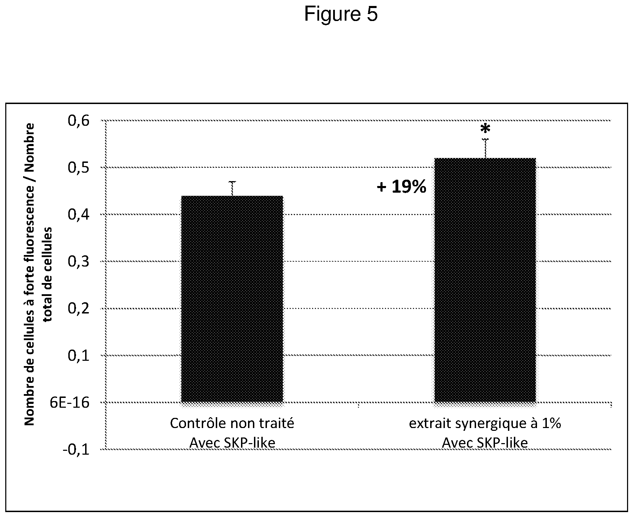

Figure 5 : Mesure de la synthèse des fibres de tropoélastine dans des équivalents de derme contenant des cellules SKP-like, après application de 1% de l'extrait synergique selon l'exemple 1. (moyenne+/- sem ; n=10-12, 3-4 photos par dermes équivalents). *: Significatif avec le test "t" de Student's - hypothèse unilatérale.

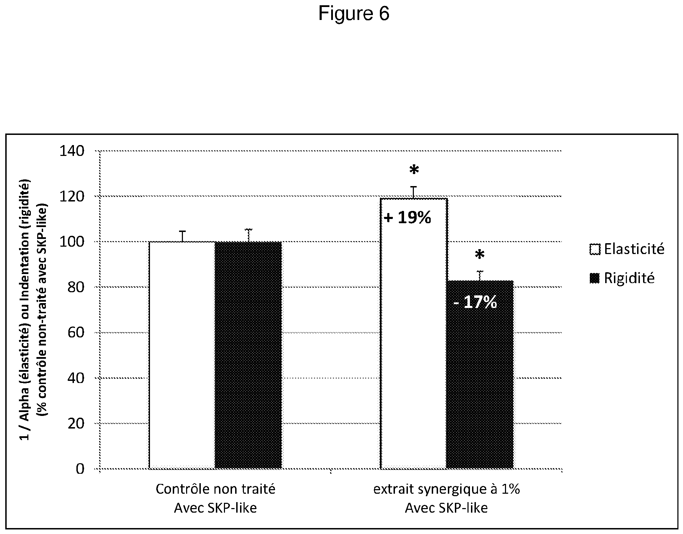

Figure 6 : Mesure de la rigidité et de l'élasticité des équivalents de derme contenant des SKP-like, après application de 1% de l'extrait synergique selon l'exemple 1. (moyenne+/- sem; n=15, 5 mesures par dermes équivalents). *: Significatif avec le test "t" de Student's - hypothèse unilatérale.

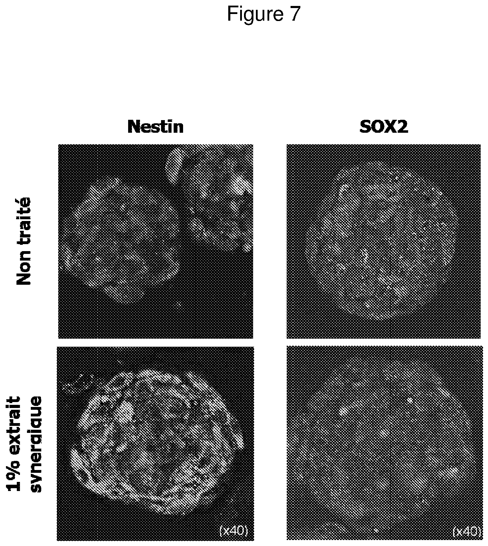

Figure 7 : Photographie des observations par microscopie optique (agrandissement *40) avant et après activation des marqueurs SOX2 et nestin, par l'extrait synergique de l'exemple 1.

Tous les résultats exprimés dans les exemples qui suivent sont statistiquement significatifs selon le test de Student ou de Dunnett (p< 0,05).

### Exemple 1 : Préparation d'un extrait synergique de Palmaria palmata et de sommités fleuries de jasmin

180 g de *Palmaria palmata,* sous forme de paillettes est mis en solution dans 4 kg d'eau et le pH est ajusté à une valeur comprise entre 4,5 et 5,5 par du HCl.

Afin d'extraire les sucres de *Palmaria palmata,* une hydrolyse à l'aide d'une hydrolase et d'une protéase est réalisée. Pour cela, 7,2 g de xylanase et 3,6 g de bromélaine sont ajoutés dans le milieu réactionnel. Le milieu réactionnel est ensuite chauffé deux heures à 55°C.

Une étape de filtration permet d'écarter le résidu solide pour ne conserver que le filtrat riche en carbohydrates. Pour cela, 10 g / kg CELATOM® (adjuvant de filtration) est ajouté et la solution est centrifugée 10 minutes à 4000 tours par minute. Après centrifugation, le solide résiduel est éliminé et le filtrat est ensuite plus ou moins clarifié par filtration sur un filtre cellulosique.

Dans une deuxième étape, 20 g de fleur de *Jasminum officinale* sont ajoutés au filtrat d'environ 3,6 kg obtenu et on réalise une macération pendant 2 heures à température ambiante.

On ajoute 10 g / kg CELATOM® au mélange et le solide est séparé du filtrat par filtration.

Le filtrat (résultant de l'hydrolyse enzymatique de *Palmaria palmata* et de la macération de sommités fleuries de jasmin) est ensuite chauffé pendant une nuit à 80 °C pour désactiver les enzymes résiduelles.

La solution est ensuite purifiée par filtration avec des filtres de porosité décroissante pour obtenir une solution limpide brillante, de couleur ambrée.

L'extrait synergique obtenu est caractérisé par une matière sèche de 28,8 ± 2,0 g / kg, une teneur en protéines est de 1,8 ± 0,5 g / kg et une teneur en sucres est de 27,3 ± 2,0 g / kg.

La solution est ensuite diluée avec de l'eau et filtrée dans des conditions stériles, puis pasteurisée à basse température (65 °C pendant une nuit) pour terminer la stérilisation.

Le produit final correspondant à l'extrait synergique actif est une solution limpide, de couleur jaune pâle caractérisé par: une matière sèche de 10 ± 3,0 g / kg, une teneur en sucres comprise entre 8 et 12 g / kg et un pH entre 4 et 5.

### Exemple 2 : Evaluation des ARNm de SOX2, Nestin et OCT4 après application d'un extrait synergique obtenu selon l'exemple 1, comparée à un extrait de Palmaria palmata de référence, et un extrait de sommités fleuries de jasmin de référence.

Le but de cette étude est de comparer la quantité d'ARNm de SOX2, de Nestin et de OCT4 exprimé par les cellules après un traitement par un extrait de jasmin de référence, par un extrait de *Palmaria palmata* de référence et par l'extrait synergique obtenu selon l'exemple 1.

Le taux d'ARNm de SOX2, Nestin ou OCT4 a été évalué par PCR quantitative (q-PCR).

Un extrait provenant des sommités fleuries de jasmin seules et un extrait provenant de *Palmaria palmata* seul ont été également réalisés dans le but d'effectuer des tests comparatifs d'efficacité biologique.

On a pris soin de préparer les extraits de référence et l'extrait synergique dans les mêmes conditions (même taux de matière végétale sèche/kg d'eau, même pH, mêmes conditions de filtration et de clarification).

### Préparation d'un extrait de jasmin de référence:

20 g de sommités fleuries de *Jasminum officinale* séchées sont placées dans 1 kg d'eau et mis à macérer 2 h à température ambiante sous agitation. Puis des filtrations successives à l'aide de filtre de porosité décroissantes (20-50 µm jusqu'à 0.3-0.5 µm) sont réalisées afin d'écarter les résidus solides et de clarifier l'extrait.

L'extrait obtenu est caractérisé par une matière sèche de 5 ± 0.5 g / kg. Par la suite l'extrait a est dilué dans de l'eau à une concentration finale de 3 ± 0.5 g / kg matière sèche. Le pH est ajusté à 4. L'extrait est ensuite soumis à une filtration stérilisante sur filtre 0.2 µm et placé à 65°C pendant une nuit pour effectuer une pasteurisation à basse température afin de terminer la stérilisation.

### Préparation d'un extrait de Palmaria palmata de référence :

50 g de *Palmaria palmata,* sous forme de paillettes sont mis en solution dans 1 kg d'eau. Une hydrolyse est réalisée à l'aide de 2 g de xylanase et 1 g de bromélaïne, ajoutés dans le milieu réactionnel. Le milieu réactionnel est ensuite chauffé deux heures à 55°C. Une étape de filtration permet d'écarter le résidu solide pour ne conserver que le filtrat riche en carbohydrates. Pour cela, 10 g / kg Celatom® (adjuvant de centrifugation) est ajouté et la solution est centrifugée 10 minutes à 4000 tours par minute. Après centrifugation, le solide résiduel est éliminé et le surnageant est ensuite clarifié par filtration sur filtre de porosité décroissante, jusqu'à 7-20 µm de porosité et placé à 80°C toute la nuit. Le jour d'après l'extrait est de nouveau filtré sur filtre de porosité décroissante, jusqu'à 0.3-0.5 µm. L'extrait obtenu est caractérisé par une matière sèche de 30 ± 2 g / kg. Par la suite l'extrait est dilué dans l'eau afin d'obtenir 20 ± 2 g / kg matière sèche. Le pH est alors ajusté à 4 - 4.5.

L'extrait est ensuite soumis à une filtration stérilisante sur filtre 0.2 µm et placé à 65 °C pendant une nuit pour effectuer une pasteurisation à basse température afin de terminer la stérilisation.

Protocole d'évaluation des ARNm: Des fibroblastes humains normaux sont traités par les différents extraits préparés précédemment et dilués à 1% vol/vol dans le milieu de culture (). En parallèle, des cultures de fibroblastes sont maintenues sans traitement, de manière à constituer un contrôle non traité. Cette culture se déroule à 37°C dans une atmosphère humidifiée contenant 5% de CO₂.

A l'issue de cette incubation, les ARN totaux sont extraits avec le RNeasy mini kit (QIAGEN, 74104) et reverse transcrits avec le High Capacity cDNA reverse-transcription kit contenant des inhibiteurs de RNAses (Applied Biosystems, 4368814). La PCR quantitative est réalisée à l'aide du thermocycler Step One Plus (Applied Biosystems). Les primers et sondes des cibles SOX2, Nestin et OCT4 ainsi que ceux du contrôle endogène 18S sont issus des Taqman Expression Assays (Applied Biosystems, Hs99999901_s1 pour 18S ; Hs01053049_s1 pour SOX2, Hs00707120_s1 pour Nestin et Hs00999632_g1 pour OCT4), dilués dans de l'eau stérile et du Master Mix (Applied Biosystems).

### Résultats :

Les résultats, tels que présentés en figure 1, montrent une augmentation statistiquement significative de respectivement 29%, 26% et 34% de l'expression des ARNm de SOX2, Nestin et OCT4 dans les fibroblastes traités avec 1% de l'extrait de *Palmaria palmata* de référence, comparé au contrôle non traité.

Aucune stimulation n'a été observée avec l'extrait de jasmin seul.

Dans le cas où les fibroblastes ont été traités par l'extrait synergique obtenu selon l'exemple 1 à 1%, les résultats montrent que les augmentations sont sensiblement plus importantes que celles observées précédemment avec l'extrait de *Palmaria palmata,* respectivement de 296 % pour de l'expression des ARNm de SOX2, de 123% pour l'expression des ARNm de Nestin et de 285% pour l'expression des ARNm de OCT4, comparé au contrôle non traité.

### Conclusion :

On observe un taux plus important d'ARNm de SOX2, Nestin et OCT4 dans les fibroblastes prétraités par l'extrait synergique obtenu selon l'exemple 1, comparé aux cellules contrôle non traitées, à l'extrait de jasmin de référence et à l'extrait d'algue de référence. L'extrait synergique obtenu selon l'exemple 1 permet d'augmenter de façon synergique les marqueurs caractéristiques du caractère « souche » des cellules dermiques.

### Exemple 3 : Mise en évidence de l'effet activateur de l'extrait synergique obtenu selon l'exemple 1 sur l'expression du Pro-collagène I et du collagène III, dans des équivalents de derme contenant des fibroblastes humains et des cellules SKPs

Le but de cette étude est de déterminer l'influence de l'extrait synergique obtenu selon l'exemple 1, sur l'expression des protéines de la matrice extracellulaire suivantes : Pro-collagène I et collagène III.

Pour cela, des marquages spécifiques ont été réalisés à partir d'équivalents de derme reconstruits constitués de collagène I bovin polymérisé, de fibroblastes humains et de cellules souches SKPs.

Protocoles des immunomarquages : Des fibroblastes humains ont été extraits à partir d'explants de peau de donneurs adultes. Les cellules SKPs utilisés dans cette expérience proviennent de peau de prépuce. Les cellules SKPs en culture sont prétraitées deux fois par jour avec l'extrait selon l'exemple 1 dilué au 1/100 dans du milieu de culture (soit 1% volume/volume), pendant 7 jours. L'extrait obtenu à l'exemple 1 ayant un poids sec d'environ 10 g/kg, l'extrait est donc testé à environ 0,01 % en poids du poids de milieu de culture. Dans ce modèle, les cellules souches sont stimulées avec l'extrait avant incorporation dans les équivalents dermiques, afin de s'assurer d'un effet direct de l'extrait (En parallèle, des cultures de cellules SKPs sont maintenues sans traitement, de manière à constituer un contrôle cellules SKPs non traité. Cette culture se déroule à 37°C dans une atmosphère humidifiée contenant 5% de CO₂.

Les équivalents de derme ont été préparés en mélangeant une solution de collagène de type I bovin dans laquelle sont introduits les cellules SKPs, cultivées sous forme de sphères, et les fibroblastes suspendus dans du milieu de culture selon une proportion respective de 1 sphère de cellules SKPs pour 10000 fibroblastes. Le mélange est soigneusement homogénéisé et distribué dans des puits de plaque 6 puits. Une polymérisation du collagène I se produit et permet la formation d'équivalent de derme. Les équivalents de derme sont traités deux fois par jour avec l'extrait synergique obtenu selon l'exemple 1, à une concentration de 1% par rapport au volume du milieu, pendant 5 jours et maintenus à 37°C dans une atmosphère humidifiée contenant 5% de CO₂.

Les échantillons sont fixés avec 10% de paraformaldéhyde et inclus en paraffine après une succession de bains d'éthanol et de xylène (Shandon).

Les immunomarquages sont réalisés sur des coupes de 4µm d'épaisseur.

Pour le Pro-collagène I, les tissues sont soumis à un démasquage aux microondes à 600W dans du tampon citrate 0.01M pH6 (Sigma), suivit d'un traitement enzymatique à la pepsine 0.25% (15min à 37°C ; Zymed, Invitrogen).

Concernant le traitement des coupes de tissues pour l'immunomarquage collagène III, un démasquage aux microondes à 600W est réalisé, suivi d'un traitement enzymatique à la trypsine 0.5% (15 min à 37°C, Zymed, Invotrogen).

Après 30 min de saturation, les coupes sont incubées en présence d'un anticorps monoclonal de rat spécifique du Pro-collagène I (Millipore), d'un anticorps polyclonal de lapin spécifique du collagène III (Rockland), puis d'un anticorps secondaire anti-rat couplé à un fluorochrome (Invitrogen) ou anti-lapin couplé à un fluorochrome (Invitrogen). Les coupes d'équivalents de derme sont alors examinées au microscope à épifluorescence (Nikon Eclipse 80i microscope).

Trois photos par condition sont analysées quantitativement avec le logiciel d'analyse d'image Volocity (Improvision).

Les analyses statistiques sont réalisées par un logiciel JMP (SAS).

### Résultats :

Les résultats, tels que présentés en figure 2, ont montré une augmentation significative de 18%, et 23% de l'expression des protéines de collagène I néo-synthétisées et de collagène III dans les équivalents de derme non traités contenant des cellules SKPs, par rapport au contrôle non traité et ne contenant pas de cellules SKPs.

Le traitement par 1 % de l'extrait synergique obtenu selon l'exemple 1, des échantillons d'équivalent de dermes contenant les cellules SKPs, a provoqué l'augmentation de 25% et 29% respectivement du Pro-collagène I et du collagène III, comparé au contrôle avec cellules SKPs et non traité.

### Conclusions :

L'application de 1% de l'extrait synergique obtenu selon l'exemple 1 dans des équivalents de derme contenant les cellules souches dermiques SKPs stimule l'expression du Pro-collagène I et du collagène III.

### Exemple 4 : Mise en évidence de l'effet activateur de l'extrait synergique obtenu selon l'exemple 1 sur la contraction dermique et l'expression du collagène III, dans des équivalents de derme contenant des fibroblastes humains et des cellules SKP-like

Wenzel et al. (Biology Open. 1:516-526, 2012) et Hill et al. (Plos One, November 2012, Vol. 7, Issue 11, e50742) ont démontré que des cellules SKP-like pouvaient être isolées après un stress au froid appliqué *in vitro* sur fibroblastes de donneurs adultes.

Le but de cette étude est de déterminer l'influence de l'extrait synergique obtenu selon l'exemple 1 sur la contraction d'équivalents de derme et sur l'expression du collagène III dans des équivalents de derme de collagène contenant des fibroblastes et des SKP-like obtenus à partir du même donneur.

La contraction des équivalents de derme est un processus lié à l'activité contractile des fibroblastes qui se lient aux microfibrilles de collagène, les orientent dans une même direction et densifient la matrice extracellulaire. Au bout de quelques jours le diamètre se stabilise. Des imunomarquages ont été réalisés à partir des équivalents de derme reconstruits pour suivre la synthèse des protéines spécifiques de la matrice extracellulaire.

Protocole: Des fibroblastes humains ont été extraits à partir d'un explant de peau de donneur adulte. Une portion de ces fibroblastes en culture a été soumise à un stress au froid (4°C, durant une nuit) afin de générer des cellules SKP-like. Les cellules présentant les mêmes caractéristiques que les cellules SKPs ainsi obtenues sont appelés ici « SKP-like ». Les cellules SKP-like utilisées dans cette expérience sont traitées deux fois par jour avec l'extrait synergique obtenu selon l'exemple 1 dilué au 1/100 dans du milieu de culture (soit 1% volume/volume), pendant 6 jours. En parallèle, des cultures de SKP-like sont maintenues sans traitement, de manière à constituer un contrôle SKP-like non traité. Cette culture se déroule à 37°C dans une atmosphère humidifiée contenant 5% de CO₂.

Les équivalents de derme ont été préparés avec un mélange de collagène de type I bovin dans lequel sont introduites les cellules SKP-like, cultivées sous forme de sphères multicellulaires. Les SKP-like et les fibroblastes ont été inclus dans une proportion de 1 sphère pour 10000 fibroblastes. Le mélange matrice de collagène I/cellules est distribué dans des puits de plaque 6 puits. Les équivalents de derme une fois polymérisés, sont cultivés sous forme flottante dans le milieu. Les équivalents de derme sont traités deux fois par jour avec l'extrait synergique obtenu selon l'exemple 1, à une concentration de 1%, pendant 5 jours et maintenus à 37°C dans une atmosphère humidifiée contenant 5% de CO₂ L'extrait de l'exemple 1 ayant un poids sec d'environ 10 g/kg, l'extrait est donc testé à environ 0,01 % en poids du poids de milieu de culture.

Chaque jour et pour 3 équivalents de derme par condition, la contraction dermique qui se traduit par une diminution du diamètre des équivalents de derme est suivi par prise de photos en culture puis quantifiée à l'aide du logiciel d'analyse d'image (Image J).

Lorsque la culture est terminée, les tissues sont fixés avec 10% de paraformaldéhyde et inclus en paraffine après une succession de bain d'éthanol et de xylène (Shandon).

L'immunomarquage a été réalisé sur des coupes de 4µm d'épaisseur.

Pour le collagène III, les coupes ont subi un démasquage microondes suivi d'un traitement enzymatique à la trypsine 0.5% (15min à 37°C, Zymed, Invotrogen). Apres 30 min de saturation, les coupes sont incubées en présence d'un anticorps polyclonal de lapin spécifique du collagène III (Rockland), puis d'un anticorps secondaire anti-lapin couplé à un fluorochrome (Invitrogen). Les coupes d'équivalents de derme sont alors examinées au microscope à épifluorescence (Nikon Eclipse 80i microscope).

Trois photos par condition sont analysées avec le logiciel d'analyse d'image Volocity (Improvision).

Les analyses statistiques sont réalisées par un logiciel JMP (SAS).

Résultats : Les résultats, tels que présentés en figure 3, ont montré une augmentation significative de 29%, de l'expression des protéines de collagène III dans les équivalents de derme traités avec 1% de l'extrait synergique obtenu selon l'exemple 1, comparé au contrôle non traité avec cellules SKP-like.

L'analyse du diamètre des équivalents de derme, tels que présentés en figure 4, a montré une augmentation très significative de la contractilité de l'équivalent de derme après 4 jours de traitement avec l'extrait synergique obtenu selon l'exemple 1, comparé au contrôle non traité avec cellules SKP-like.

Conclusions : L'application de 1% de l'extrait synergique obtenu selon l'exemple 1 dans des équivalents de derme contenant les cellules « souches » dermiques (Cellules SKP-like) obtenus à partir de fibroblastes de donneur adulte améliore la contractilité du tissue et stimule l'expression du collagène III.

### Exemple 5 : Mise en évidence de l'effet activateur de l'extrait synergique obtenu selon l'exemple 1 sur la synthèse des fibres de tropoélastine, dans des équivalents de derme contenant des fibroblastes humains et des cellules SKP-like

Le but de cette étude est de déterminer l'influence de l'extrait synergique obtenu selon l'exemple 1 sur la synthèse de fibres de tropoélastine dans des équivalents de derme de collagène contenant des fibroblastes et des SKP-like obtenus à partir du même donneur. Un immunomarquage a été réalisé à partir des équivalents de derme reconstruits pour suivre la synthèse des fibres de tropoélastine à partir des fibroblastes.

Protocole: Des équivalents de derme de collagène contenant des fibroblastes et des SKP-like obtenus à partir du même donneur ont été préparés suivant le même protocole que spécifié dans l'exemple 5 à l'exception des traitements. En effet, les cellules SKP-like utilisées dans cette expérience sont traitées deux fois par jour avec l'extrait synergique obtenu selon l'exemple 1 dilué au 1/100 dans du milieu de culture (soit 1% volume/volume). , pendant 5 jours. Les équivalents de derme sont traités deux fois par jour avec l'extrait synergique obtenu selon l'exemple 1 à une concentration de 1%, pendant 6 jours. L'extrait obtenu à l'exemple 1 ayant un poids sec d'environ 10 g/kg, l'extrait est donc testé à environ 0,01 % en poids du poids de milieu de culture.

Lorsque la culture est terminée, les tissues sont fixés avec 10% de paraformaldéhyde et inclus en paraffine après une succession de bain d'éthanol et de xylène (Shandon).

L'immunomarquage a été réalisé sur des coupes de 4µm d'épaisseur.

Les coupes ont subi un démasquage par traitement enzymatique à la trypsine 0.5% (15 min à 37°C, Zymed, Invotrogen). Apres 30 min de saturation, les coupes sont incubées en présence d'un anticorps polyclonal de lapin spécifique de la tropoélastine (Abcam), puis d'un anticorps secondaire anti-lapin couplé à un fluorochrome (Invitrogen). Les coupes d'équivalents de derme sont alors examinées au microscope à épifluorescence (Nikon Eclipse 80i microscope).

Dix à douze photos par conditions sont analysées. Une numération du nombre de cellules à forte fluorescence ainsi que du nombre de cellules totales sont faites.

Les analyses statistiques sont réalisées par un logiciel JMP (SAS).

Résultats : Les résultats, tels que présentés en figure 5, ont montré une augmentation significative de 19%, de la synthèse des fibres de tropoélastine dans les équivalents de derme traités avec 1% de l'extrait synergique obtenu selon l'exemple 1, comparé au contrôle non traité avec cellules SKP-like.

Conclusions : L'application de 1% de l'extrait synergique obtenu selon l'exemple 1 dans des équivalents de derme contenant les cellules « souches » dermiques (cellules SKP-like) obtenus à partir de fibroblastes de donneur adulte, améliore la synthèse des fibres élastiques.

### Exemple 6 : Mise en évidence de l'effet activateur de l'extrait synergique obtenu selon l'exemple 1 sur les propriétés élastiques des équivalents de derme contenant des fibroblastes humains et des cellules SKP-like

Le but de cette étude est de déterminer l'influence de l'extrait synergique obtenu selon l'exemple 1 sur les propriétés élastiques d'équivalents de derme de collagène contenant des fibroblastes et des cellules SKP-like obtenus à partir du même donneur. Le ballistomètre de référence BLS780 (Monaderm) est un appareil permettant de déterminer l'élasticité et la rigidité de la peau. il est fourni avec un logiciel qui paramètre automatiquement les mesures.

Protocole: Des équivalents de derme de collagène contenant des fibroblastes et des SKP-like obtenus à partir du même donneur ont été préparés suivant le même protocole que spécifié dans l'exemple 4 à l'exception des traitements. En effet, les cellules SKP-like utilisées dans cette expérience sont traitées deux fois par jour avec l'extrait synergique obtenu selon l'exemple 1 dilué au 1/100 dans du milieu de culture (soit 1% volume/volume, pendant 5 jours. Les équivalents de derme sont traités deux fois par jour avec l'extrait synergique obtenu selon l'exemple 1 à une concentration de 1%, pendant 10 jours). L'extrait obtenu à l'exemple 1 ayant un poids sec d'environ 10 g/kg, l'extrait est donc testé à environ 0,01 % en poids du poids de milieu de culture.

Méthodologie : Une sonde est posée sur l'équivalent de derme. Une bille est libérée de la sonde et pénètre avec une force constante, prédéterminée par l'appareil, et rebondie à la surface de la peau. Elle réalise alors plusieurs oscillations avant de se stabiliser. La profondeur de pénétration de la bille à sa libération (nommée indentation) permet de mesurer la rigidité de la peau. En effet plus la bille s'enfonce profondément plus le tissu est mou donc avec une rigidité moins grande. Le second critère est la pente de la courbe reliant tous les sommets des pics (nommé alpha) permet de déterminer l'élasticité de la peau. Dans le cas d'une peau bien élastique la bille réalise plus de rebonds et donc la pente est faible.

Résultats : Les résultats, tels que présentés en figure 6, ont montré une augmentation significative de 19% de l'élasticité des équivalents de derme traités avec 1% de l'extrait synergique obtenu selon l'exemple 1, comparé au contrôle non traité avec cellules SKP-like. En parallèle, une diminution significative de 17% de la rigidité des équivalents de derme a été observée comparé au contrôle.

Conclusions : L'application de 1% de l'extrait synergique obtenu selon l'exemple 1 dans des équivalents de derme contenant les cellules « souches » dermiques (cellules SKP-like) obtenus à partir de fibroblastes de donneur adulte améliore les propriétés élastiques et la souplesse d'équivalents de derme.

### Exemple 7 : Exemples de compositions cosmétiques contenant l'extrait synergique de l'invention

### A- SERUM

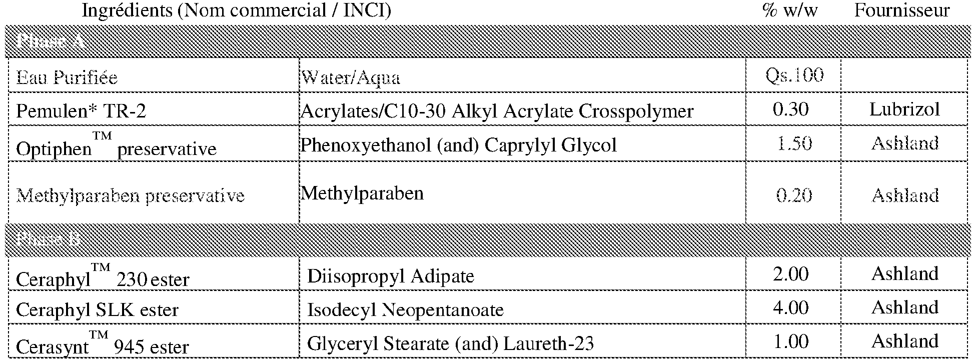

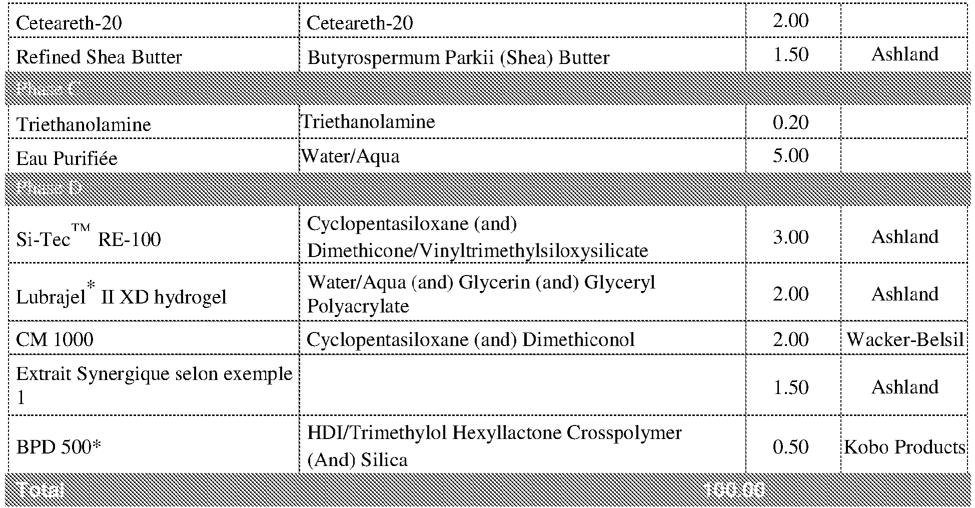

### Protocole de préparation :

Ajouter l'eau dans le récipient principal. Chauffer sous homogénéisation douce.

Verser lentement le Pemulen TR-2 dans l'eau et mélanger jusqu'à réhydratation complète. Maintenir la température entre 70°et 75°C.

Lorsque le polymère est complètement mélangé, sans aucun grumeaux, ajouter les conservateurs un à un en mélangeant.

Dans un deuxième récipient, mélanger la Phase B et chauffer à 75°C
Ajouter la Phase B au récipient principal en homogénéisant.

Refroidir à 60°-65°C et ajouter la Phase C pré-mélangée.

Refroidir à 40°-45°C et ajouter les ingrédients de la Phase D, un à un, en mélangeant entre chaque addition.

Refroidir sous agitation jusqu'à température ambiante (25°C).

### B-Crème de nuit

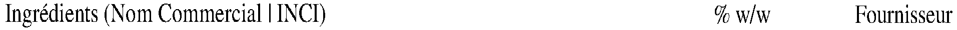

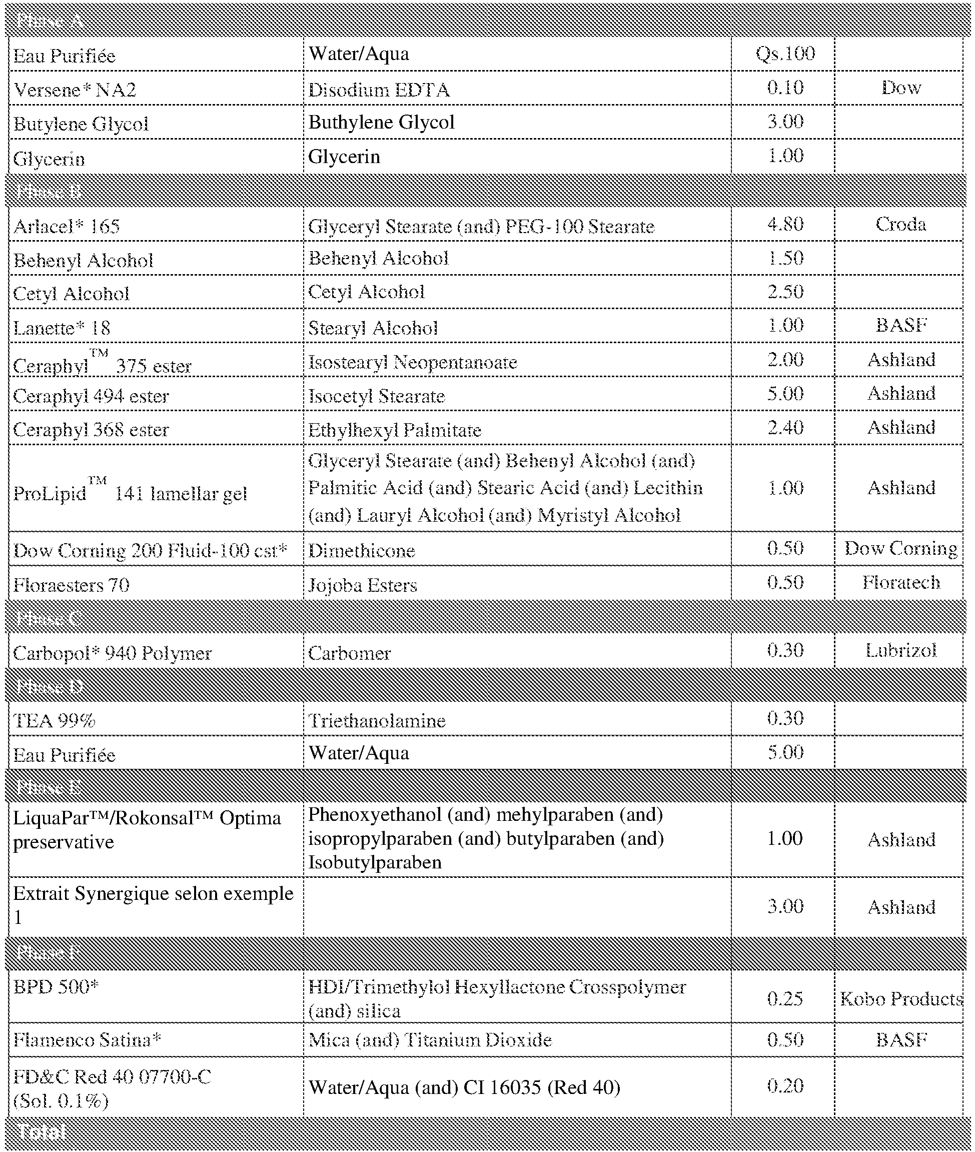

### Protocole :

Ajouter l'eau dans le récipient principal sous homogénéisation douce. Ajouter le reste des ingrédients de la Phase A un par un et chauffer à 70°-75°C en mélangeant.

Dans un 2ème récipient, mélanger les ingrédients de la Phase B et chauffer à 75°C en mélangeant.

Ajouter la Phase B (70°-75°C) à la Phase A (70°-75°C) sous forte agitation (10minutes) utilisant. Commencer à refroidir.

A 60°-65°C, saupoudrer la Phase C dans le mélange. Mélanger bien pour obtenir une hydratation complète du polymère (éventuellement augmenter la vitesse de mélange).

Pré-mélanger la Phase D; ajouter au mélange et mélanger le tout soigneusement pour obtenir un mélange uniforme. Le mélange s'épaissie. Continuer à refroidir.

Refroidir sous agitation lente jusqu' à 40 °C. Ajouter les ingrédients de la phase E un à un en agitant bien entre chaque ajout.

A température ambiante, ajouter la Phase F. Bien mélanger. Stop à 25°C

### C- Crème détoxifiante

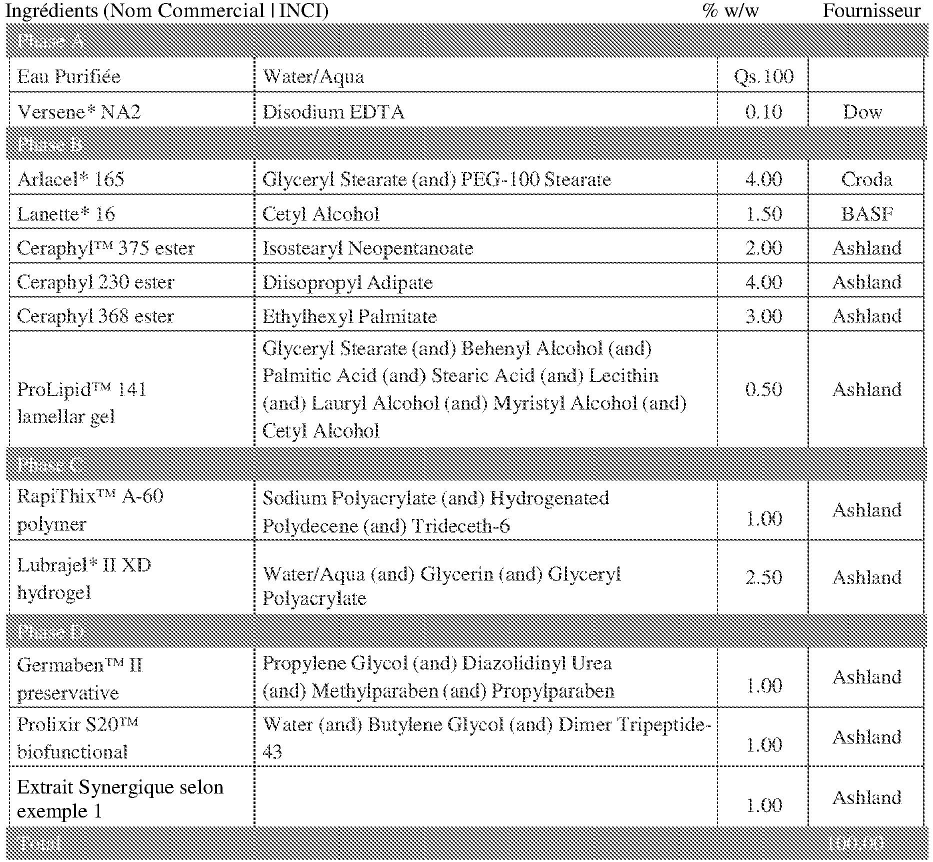

### Protocole :

Ajouter l'eau dans le récipient principal sous homogénéisation douce. Ajouter le Versene et mélanger jusqu'à dissolution complète. Chauffer à 70°-75°C.

Dans un 2ème récipient, mélanger les ingrédients de la Phase B et chauffer à 70°C-75°C en mélangeant.

A 75°C, ajouter la Phase B à la Phase A sous forte pendant 10 minutes. Commencer à refroidir.

Refroidir à 60°-65°C et ajouter les ingrédients de la Phase C un à un en mélangeant entre chaque ajout.

Refroidir à 40°-45°C et ajouter les ingrédients de la Phase D, un à un, en mélangeant entre chaque ajout.

Refroidir sous lente agitation jusqu'à température ambiante. Stop à 25°C.

### Exemple 8 : Mise en évidence de l'effet activateur de l'extrait synergique obtenu selon l'exemple 1 sur l'expression protéique des marqueurs SOX2, OCT4 et nestin dans des cellules SKP-like

Le but de cette étude est de déterminer l'influence de l'extrait synergique obtenu selon l'exemple 1, sur l'expression de protéines caractéristiques des cellules souches : SOX2, OCT4 et nestin.

Pour cela, des marquages spécifiques ont été réalisés sur des culots de SKPs-like inclus en paraffine.

Protocoles des immunomarquages : Des SKPs-like ont été isolés à partir de fibroblastes humains extraits à partir d'explants de peau de donneurs adultes. Les cellules SKPs-like sont traitées deux fois par jour avec l'extrait selon l'exemple 1 dilué au 1/100 dans du milieu de culture (soit 1% volume/volume), pendant 2 jours. L'extrait obtenu à l'exemple 1 ayant un poids sec d'environ 10 g/kg, l'extrait est donc testé à environ 0,01 % en poids du poids de milieu de culture. Cette culture se déroule à 37°C dans une atmosphère humidifiée contenant 5% de CO₂.

A la fin de la culture, les SKPs-like sont préparés en culot cellulaire, fixés avec 10% de paraformaldéhyde et inclus en paraffine après une succession de bains d'éthanol et de xylène (Shandon).

Les immunomarquages sont réalisés sur des coupes de 4µm d'épaisseur.

Pour les trois immunomarquages, les coupes sont soumises à un démasquage aux micro-ondes à 600W dans du tampon citrate 0.01M pH6 (Sigma). Après 30 min de saturation, les coupes sont incubées en présence d'un anticorps monoclonal de souris spécifique de nestin (abcam), d'un anticorps polyclonal de lapin spécifique d'OCT4 ou de SOX2 (Abcam), puis d'un anticorps secondaire anti-souris couplé à un fluorochrome (Invitrogen) ou anti-lapin couplé à un fluorochrome (Invitrogen). Les coupes de SKPs-like sont alors examinées au microscope à épifluorescence (Nikon Eclipse 80i microscope).

### Résultats :

Les observations microscopiques (agrandissement *40) ont montré une augmentation de l'intensité des marquages SOX2, OCT4 et nestin dans les conditions où l'extrait synergique obtenu selon l'exemple 1 a été appliqué, comparé au non traité. La Figure 7 reproduit les clichés obtenus pour les marquages SOX2 et nestin.

### Conclusions :

L'application de 1% de l'extrait synergique obtenu selon l'exemple 1 dans des SKPs-like stimule l'expression des marqueurs de cellules souches SOX2, OCT4 et nestin.

## Revendications

1. Extrait synergique de l'algue *Palmaria palmata* et de sommités fleuries d'une plante du genre *Jasminum,* lequel extrait est susceptible d'être obtenu par un procédé comprenant i) une étape de préparation d'un extrait aqueux de l'algue *Palmaria palmata* suivie ii) d'une étape de macération des sommités fleuries d'une plante du genre *Jasminum* dans ledit extrait aqueux, le ratio massique entre le poids sec de l'algue et le poids sec des sommités fleuries, toutes deux utilisées comme matière première pour préparer l'extrait synergique, étant compris entre 40/60 et 95/5.

2. Extrait selon la revendication 1, **caractérisé en ce que** le ratio massique entre le poids sec de l'algue et le poids sec des sommités fleuries est compris entre 8/1 et 10/1.

3. Extrait selon l'une des revendications précédentes, **caractérisé en ce que** la plante du genre *Jasminum* est choisie parmi les espèces *Jasminum grandiflorum, Jasminum officinale, Jasminum odoratissimum, Jasminum sambac, Jasminum auriculatum, Jasminum flexile,* préférentiellement *Jasminum officinale.*

4. Extrait selon l'une des revendications précédentes, **caractérisé en ce que** les sommités fleuries sont entières et séchées.

5. Extrait selon l'une des revendications précédentes, **caractérisé en ce que**, à l'étape i), l'extrait aqueux de *Palmaria palmata* est obtenu par une hydrolyse enzymatique à l'aide d'une carbohydrase et d'une endoprotéase, puis par filtration et/ou centrifugation.

6. Extrait selon la revendication 5, **caractérisé en ce que** la carbohydrase est la xylanase, utilisée préférentiellement à une concentration comprise entre 2 et 6% en poids du poids sec de l'algue, et préférentiellement de 4% et que l'endoprotéase est la bromélaïne utilisée à une concentration comprise entre 1 à 3% et préférentiellement de 2%, en poids du poids sec de l'algue.

7. Extrait selon l'une des revendications précédentes, **caractérisé en ce qu'**il contient une teneur en matière sèche comprise entre 26,8 et 30,8 g/kg, une teneur en protéines comprise entre 1,3 et 2,3 g/kg et une teneur en sucres comprise entre 25,3 et 29,3 g/kg.

8. Extrait selon la revendication 7, **caractérisé en ce que**, à l'issue de l'étape ii), il est dilué dans un ou plusieurs solvants physiologiquement acceptables choisis parmi l'eau, le glycérol, l'éthanol, le propanediol, le butylène glycol, le dipropylène glycol, les diglycols éthoxylés ou propoxylés, les polyols cycliques ou tout mélange de ces solvants.

9. Procédé d'obtention d'un extrait synergique de *Palmaria palmata* et de sommités fleuries d'une plante du genre *Jasminum* selon l'une des revendications 1 à 8, **caractérisé en ce qu'**il comprend les étapes suivantes selon lesquelles :
a) on met en solution dans de l'eau une quantité de *Palmaria palmata* séchées et finement broyées, dans un rapport massique eau / *Palmaria palmata* compris entre 10/1 et 50/1, préférentiellement entre 20/1 et 40/1.
b) on hydrolyse la solution aqueuse de *Palmaria palmata* par une carbohydrase et/ou une endoprotéase à un pH compris entre 3 et 6, préférentiellement entre 4 et 5,5, encore plus préférentiellement entre 4 et 4,5, à une température comprise entre 40 et 80°C, préférentiellement entre 50 et 60°C, encore plus préférentiellement de 55°C, pendant un temps d'au moins 1 heure, préférentiellement 2 heures;
c) après ajout éventuel d'un adjuvant de filtration et d'une centrifugation, on obtient un extrait aqueux de *Palmaria palmata* ;
d) on fait macérer pendant un temps d'au moins 2 heures et au plus 4 heures à température ambiante des sommités fleuries séchées d'une plante du genre *Jasminum* dans l'extrait aqueux de *Palmaria palmata* obtenu à l'étape c) ; le ratio massique entre le poids sec de l'algue et le poids sec des sommités fleuries est compris entre 40/60 et 95/5.
e) on filtre le macérât ainsi obtenu à l'étape d) pour récupérer un extrait de *Palmaria palmata* et de sommités fleuries d'une plante du genre *Jasminum* que l'on chauffe pendant au moins 2 heures et jusqu'à 24 heures et préférentiellement pendant 12 heures ou une nuit, à une température comprise entre 40 et 90°C, préférentiellement à 80°C pour désactiver les enzymes carbohydrase et endoprotéase ; et
f) on purifie éventuellement par filtration pour obtenir l'extrait synergique de *Palmaria palmata* et de sommités fleuries d'une plante du genre *Jasminum.*

10. Composition cosmétique comprenant, dans un solvant physiologiquement acceptable, un extrait synergique de *Palmaria palmata* et de sommités fleuries d'une plante du genre *Jasminum* selon l'une des revendications 1 à 8, ou obtenu selon le procédé de la revendication 9.

11. Composition selon la revendication 10, **caractérisée en ce qu'**elle comprend l'extrait synergique de *Palmaria palmata* et de sommités fleuries d'une plante du genre *Jasminum* à une concentration comprise entre 0,0001% et 20% en poids sec du poids total de la composition, et préférentiellement à une concentration comprise entre 0,05% et 5% en poids sec du poids total de la composition.

12. Composition selon l'une des revendications 10 ou 11, **caractérisée en ce qu'**elle comprend, en outre, au moins un agent actif additionnel choisi parmi la vitamine A, l'acide rétinoïque, le rétinol, la vitamine B3, la vitamine B5, la vitamine B6, la vitamine B12, la vitamine C, la vitamine E, la vitamine F, la vitamine H, la vitamine K, la vitamine PP, le coenzyme Q10, les inhibiteurs de métalloproteinase, les acides aminés, la carnitine, la carnosine, la taurine, les peptides naturels ou de synthèse, les extraits peptidiques végétaux, les extraits de levures, les extraits *d'Artemia salina*, les phytostérols d'origine synthétique ou naturelle, l'acide salicylique, les oligosaccharides, les polysaccharides, les sucres aminés, les polyphénols, les flavonoïdes, les lipides, les phospholipides, les huiles d'origine animale, les huiles végétales, les huiles végétales éthoxylées, les écrans UV et les filtres solaires.

13. Utilisation cosmétique d'un extrait synergique de *Palmaria palmata* et de sommités fleuries d'une plante du genre *Jasminum* selon l'une des revendications 1 à 8 ou d'une composition selon l'une des revendications 10 à 12, pour lutter contre les signes de vieillissement de la peau en favorisant le maintien du caractère « souche » des cellules souches adultes dermiques (SKPs).

14. Utilisation selon la revendication 13, pour améliorer l'élasticité, la souplesse et/ou la fermeté de la peau.

## Patentansprüche

1. Synergistischer Extrakt der *Palmaria-palmata*-Alge und von Blütenspitzen einer Pflanze der Gattung *Jasminum,* wobei der Extrakt dazu geeignet ist, durch ein Verfahren erhalten zu werden, das i) einen Schritt der Herstellung eines wässrigen Extrakts der *Palmaria-palmata*-Alge umfasst, gefolgt von ii) einem Schritt der Mazeration von Blütenspitzen einer Pflanze der Gattung *Jasminum* in dem wässrigen Extrakt, wobei das Massenverhältnis zwischen dem Trockengewicht der Alge und dem Trockengewicht der Blütenspitzen, wobei beide als Rohstoff zur Herstellung des synergistischen Extrakts verwendet werden, zwischen 40/60 und 95/5 liegt.

2. Extrakt nach Anspruch 1, **dadurch gekennzeichnet, dass** das Massenverhältnis zwischen dem Trockengewicht der Alge und dem Trockengewicht der Blütenspitzen zwischen 8/1 und 10/1 liegt.

3. Extrakt nach einem der vorhergehenden Ansprüche, **dadurch gekennzeichnet, dass** die Pflanze der Gattung *Jasminum* aus den Spezies *Jasminum grandiflorum*, *Jasminum officinale, Jasminum odoratissimum, Jasminum sambac, Jasminum auriculatum*, *Jasminum flexile* ausgewählt ist, vorzugsweise *Jasminum officinale* ist.

4. Extrakt nach einem der vorhergehenden Ansprüche, **dadurch gekennzeichnet, dass** die Blütenspitzen ganz und getrocknet sind.

5. Extrakt nach einem der vorhergehenden Ansprüche, **dadurch gekennzeichnet, dass** im Schritt i) der wässrige Extrakt von *Palmaria palmata* durch eine enzymatische Hydrolyse mithilfe einer Carbohydrase und einer Endoprotease und dann durch Filtration und/oder Zentrifugation erhalten wird.

6. Extrakt nach Anspruch 5, **dadurch gekennzeichnet, dass** die Carbohydrase Xylanase ist, die vorzugsweise in einer Konzentration verwendet wird, die zwischen 2 und 6 Gew.-% des Trockengewichts der Alge und vorzugsweise bei 4 % liegt, und die Endoprotease Bromelain ist, das in einer Konzentration verwendet wird, die zwischen 1 und 3 Gew.-% und vorzugsweise bei 2 Gew.-% des Trockengewichts der Alge liegt.

7. Extrakt nach einem der vorhergehenden Ansprüche, **dadurch gekennzeichnet, dass** er einen Trockensubstanzgehalt, der zwischen 26,8 und 30,8 g/kg liegt, einen Proteingehalt, der zwischen 1,3 und 2,3 g/kg liegt, und einen Zuckergehalt, der zwischen 25,3 und 29,3 g/kg liegt, enthält.

8. Extrakt nach Anspruch 7, **dadurch gekennzeichnet, dass** er zum Ende des Schritts ii) in einem oder mehreren physiologisch akzeptablen Lösungsmitteln verdünnt wird, das/die aus Wasser, Glycerin, Ethanol, Propandiol, Butylenglykol, Dipropylenglykol, ethoxylierten oder propoxylierten Diglykolen, cyclischen Polyolen oder einem beliebigen Gemisch dieser Lösungsmittel ausgewählt ist/sind.

9. Verfahren zum Erhalten eines synergistischen Extrakts von *Palmaria palmata* und von Blütenspitzen einer Pflanze der Gattung *Jasminum* nach einem der Ansprüche 1 bis 8, **dadurch gekennzeichnet, dass** es die folgenden Schritte umfasst, in denen:
a) eine Menge von getrocknetem und fein gemahlenem *Palmaria palmata* in Wasser in einem Wasser/*Palmaria-palmata*-Massenverhältnis, das zwischen 10/1 und 50/1, vorzugsweise zwischen 20/1 und 40/1 liegt, in Lösung gebracht wird,
b) die wässrige Lösung von *Palmaria palmata* durch eine Carbohydrase und/oder eine Endoprotease bei einem pH-Wert, der zwischen 3 und 6, vorzugsweise zwischen 4 und 5,5, noch bevorzugter zwischen 4 und 4,5 liegt, und bei einer Temperatur, die zwischen 40 und 80 °C, vorzugsweise zwischen 50 und 60 °C, noch bevorzugter bei 55 °C liegt, für eine Zeit von mindestens 1 Stunde, vorzugsweise 2 Stunden hydrolysiert wird,
c) nach einer optionalen Zugabe eines Filtrations- und Zentrifugationshilfsmittels ein wässriger Extrakt von *Palmaria palmata* erhalten wird,
d) für eine Zeit von mindestens 2 Stunden und höchstens 4 Stunden bei Umgebungstemperatur getrocknete Blütenspitzen einer Pflanze der Gattung *Jasminum* in dem im Schritt c) erhaltenen wässrigen Extrakt von *Palmaria palmata* mazeriert werden, wobei das Massenverhältnis zwischen dem Trockengewicht der Alge und dem Trockengewicht der Blütenspitzen zwischen 40/60 und 95/5 liegt,
e) das so im Schritt d) erhaltene Mazerat filtriert wird, um einen Extrakt von *Palmaria palmata* und von Blütenspitzen einer Pflanze der Gattung *Jasminum* zu gewinnen, der für mindestens 2 Stunden und bis zu 24 Stunden und vorzugsweise für 12 Stunden oder eine Nacht bei einer Temperatur, die zwischen 40 und 90 °C, vorzugsweise bei 80 °C liegt, erhitzt wird, um die Enzyme Carbohydrase und Endoprotease zu deaktivieren, und
f) optional durch Filtration aufgereinigt wird, um den synergistischen Extrakt von *Palmaria palmata* und von Blütenspitzen einer Pflanze der Gattung *Jasminum* zu erhalten.

10. Kosmetische Zusammensetzung, die in einem physiologisch akzeptablen Lösungsmittel einen synergistischen Extrakt von *Palmaria palmata* und von Blütenspitzen einer Pflanze der Gattung *Jasminum* nach einem der Ansprüche 1 bis 8 umfasst oder gemäß dem Verfahren nach Anspruch 9 erhalten wird.

11. Zusammensetzung nach Anspruch 10, **dadurch gekennzeichnet, dass** sie den synergistischen Extrakt von *Palmaria palmata* und von Blütenspitzen einer Pflanze der Gattung *Jasminum* in einer Konzentration, die zwischen 0,0001 Trockengew.-% und 20 Trockengew.-% des Gesamtgewichts der Zusammensetzung liegt, und vorzugsweise in einer Konzentration, die zwischen 0,05 Trockengew.-% und 5 Trockengew.-% des Gesamtgewichts der Zusammensetzung liegt, umfasst.

12. Zusammensetzung nach einem der Ansprüche 10 oder 11, **dadurch gekennzeichnet, dass** sie ferner mindestens einen zusätzlichen Wirkstoff umfasst, der aus Vitamin A, Retinsäure, Retinol, Vitamin B3, Vitamin B5, Vitamin B6, Vitamin B12, Vitamin C, Vitamin E, Vitamin F, Vitamin H, Vitamin K, Vitamin PP, Koenzym Q10, Metalloproteinasehemmern, Aminosäuren, Carnitin, Carnosin, Taurin, natürlichen oder synthetischen Peptiden, pflanzlichen Peptidextrakten, Hefeextrakten, Extrakten von *Artemia salina,* Phytosterolen mit synthetischem oder natürlichem Ursprung, Salicylsäure, Oligosacchariden, Polysacchariden, Aminozuckern, Polyphenolen, Flavonoiden, Lipiden, Phospholipiden, Ölen mit tierischem Ursprung, pflanzlichen Ölen, ethoxylierten pflanzlichen Ölen, UV-Schutzmitteln und Sonnenschutzmitteln ausgewählt ist.

13. Kosmetische Verwendung eines synergistischen Extrakts von *Palmaria palmata* und von Blütenspitzen einer Pflanze der Gattung *Jasminum* nach einem der Ansprüche 1 bis 8 oder einer Zusammensetzung nach einem der Ansprüche 10 bis 12 zur Bekämpfung von Anzeichen einer Alterung der Haut durch Fördern der Aufrechterhaltung der "Stamm"-Eigenschaft von adulten Hautstammzellen (SKP).

14. Verwendung nach Anspruch 13 zur Verbesserung der Elastizität, der Geschmeidigkeit und/oder der Straffheit der Haut.

## Claims

1. A synergistic extract of the alga *Palmaria palmata* and of flower heads of a plant of the genus *Jasminum,* said extract being obtainable by a method comprising i) a step of preparing an aqueous extract of the alga *Palmaria palmata* followed by ii) a step of maceration of the flower heads of a plant of the genus *Jasminum* in said aqueous extract, the weight ratio of the dry weight of the alga to the dry weight of the flower heads, both used as raw material for preparing the synergistic extract, being between 40/60 and 95/5.

2. The extract as claimed in claim 1, **characterized in that** the weight ratio of the dry weight of the alga to the dry weight of the flower heads is between 8/1 and 10/1.

3. The extract according to one of the preceding claims, **characterized in that** the plant of the genus *Jasminum* is selected from the species *Jasminum grandiflorum, Jasminum officinale, Jasminum odoratissimum, Jasminum sambac, Jasminum auriculatum, Jasminum flexile,* preferably *Jasminum officinale.*

4. The extract according to one of the preceding claims, **characterized in that** the flower heads are whole and dried flower heads.

5. The extract according to one of the preceding claims, **characterized in that**, in step i), the aqueous extract of *Palmaria palmata* is obtained by enzymatic hydrolysis using a carbohydrase and an endoprotease, and then filtration and/or centrifugation.

6. The extract as claimed in claim 5, **characterized in that** the carbohydrase is xylanase, preferably used at a concentration between 2 and 6 wt% of the dry weight of the alga, and preferably 4%, and **in that** the endoprotease is bromelain used at a concentration between 1 and 3% and preferably 2%, by weight of the dry weight of the alga.

7. The extract according to one of the preceding claims, **characterized in that** it has a dry matter content between 26.8 and 30.8 g/kg, a content of proteins between 1.3 and 2.3 g/kg and a content of sugars between 25.3 and 29.3 g/kg.

8. The extract as claimed in claim 7, **characterized in that**, at the end of step ii), it is diluted in one or more physiologically acceptable solvents selected from water, glycerol, ethanol, propanediol, butylene glycol, dipropylene glycol, ethoxylated or propoxylated diglycols, cyclic polyols or any mixture of these solvents.

9. A method of obtaining a synergistic extract of *Palmaria palmata* and of flower heads of a plant of the genus *Jasminum* as claimed in one of claims 1 to 8, **characterized in that** it comprises the following steps, according to which:
a) an amount of dried and finely ground *Palmaria palmata alga* is dissolved in water in a weight ratio of water to *Palmaria palmata* between 10/1 and 50/1, preferably between 20/1 and 40/1.
b) the aqueous solution of *Palmaria palmata* is hydrolyzed with a carbohydrase and/or an endoprotease at a pH between 3 and 6, preferably between 4 and 5.5, even more preferably between 4 and 4.5, at a temperature between 40 and 80°C, preferably between 50 and 60°C, even more preferably 55°C, for a time of at least 1 hour, preferably 2 hours;
c) after optional addition of a filter aid and centrifugation, an aqueous extract of *Palmaria palmata* is obtained;
d) dried flower heads of a plant of the genus *Jasminum* are macerated for a time of at least 2 hours and at most 4 hours at ambient temperature in the aqueous extract of *Palmaria palmata* obtained in step c); the weight ratio of the dry weight of the alga to the dry weight of the flower heads is between 40/60 and 95/5.
e) the macerated product thus obtained in step d) is filtered to recover an extract of *Palmaria palmata* and of flower heads of a plant of the genus *Jasminum,* which is heated for at least 2 hours and for up to 24 hours and preferably for 12 hours or overnight, at a temperature between 40 and 90°C, preferably at 80°C to deactivate the carbohydrase and endoprotease enzymes; and
f) it is purified optionally by filtration to obtain the synergistic extract of *Palmaria palmata* and of flower heads of a plant of the genus *Jasminum.*

10. A cosmetic composition comprising, in a physiologically acceptable solvent, a synergistic extract of *Palmaria palmata* and of flower heads of a plant of the genus *Jasminum* as claimed in one of claims 1 to 8, or obtained by the method of claim 9.

11. The composition as claimed in claim 10, **characterized in that** it comprises the synergistic extract of *Palmaria palmata* and of flower heads of a plant of the genus *Jasminum* at a concentration between 0.0001% and 20 % dry weight of the total weight of the composition, and preferably at a concentration between 0.05% and 5 % dry weight of the total weight of the composition.

12. The composition as claimed in one of claims 10 or 11, **characterized in that** it further comprises at least one additional active agent selected from vitamin A, retinoic acid, retinol, vitamin B3, vitamin B5, vitamin B6, vitamin B12, vitamin C, vitamin E, vitamin F, vitamin H, vitamin K, vitamin PP, coenzyme Q10, metalloproteinase inhibitors, amino acids, carnitine, carnosine, taurine, natural or synthetic peptides, vegetable peptide extracts, yeast extracts, extracts of *Artemia salina,* phytosterols of synthetic or natural origin, salicylic acid, oligosaccharides, polysaccharides, amino sugars, polyphenols, flavonoids, lipids, phospholipids, oils of animal origin, vegetable oils, ethoxylated vegetable oils, UV screens and sun filters.

13. A cosmetic use of a synergistic extract of *Palmaria palmata* and of flower heads of a plant of the genus *Jasminum* as claimed in one of claims 1 to 8 or of a composition as claimed in one of claims 10 to 12, for combating the signs of aging skin by favoring maintenance of the "stem" character of the adult dermal stem cells (SKPs).

14. The use as claimed in claim 13, for improving elasticity, flexibility and/or firmness of a skin.
